# EUROPÄISCHE PATENTSCHRIFT

(11) **EP 0 486 580 B1**
(45) Veröffentlichungstag und Bekanntmachung des Hinweises auf die Patenterteilung: **06.11.1996**
(21) Anmeldenummer: 90912431.5
(22) Anmeldetag: 18.08.1990
(51) Int. Cl.: C12Q 1/68, B01D 57/02, G01N 27/26

(54) **VERFAHREN UND VORRICHTUNG ZUR TRENNUNG UND DETEKTION VON KOMPONENTEN EINES STOFFGEMISCHES DURCH TEMPERATURGRADIENTEN-GELELEKTROPHORESE**
PROCESS AND DEVICE FOR SEPARATING AND DETECTING CONSTITUENTS OF A MIXTURE OF SUBSTANCES BY TEMPERATURE GRADIENT GEL ELECTROPHORESIS
PROCEDE ET DISPOSITIF PERMETTANT DE SEPARER ET DE DETECTER LES ELEMENTS D'UN MELANGE DE SUBSTANCES PAR ELECTROPHORESE A GEL A GRADIENT DE TEMPERATURE

(30) Priorität: 19.08.1989 DE 3927467; 06.03.1990 DE 4006974
(43) Veröffentlichungstag der Anmeldung: 27.05.1992
(73) Patentinhaber: QIAGEN GmbH, 40724 Hilden (DE)
(72) Erfinder: HENCO, Karsten, D-4006 Erkrath 2 (DE); RIESNER, Detlev, D-4000 Düsseldorf 12 (DE); STEGER, Gerhard, D-4000 Düsseldorf 1 (DE)
(74) Vertreter: Werner, Hans-Karsten, Dr.Dipl.-Chem.
(86) Internationale Anmeldenummer: EP9001366
(87) Internationale Veröffentlichungsnummer: WO9102815

(56) Entgegenhaltungen:
- EP-A- 0 198 207
- EP-A- 0 253 303
- EP-A- 0 318 273
- US-A- 4 671 870
- Electrophoresis, Band 10(5-6), 19. Juni 1989, VCH, (Weinheim, DE), D. RIESNER et al.: "Temperature-Gradient Gel Electrophoresis of Nucleic Acids: Analysis of Conformational Transitions, Sequence Variations, and Protein-Nucleic Acid Interactions", seiten 377-389 siehe den ganzen artikel; Insbesondere die Zusammenfassung; seite 378, spalte 1, zeile 54 - spalte 2, zeile 14 in der anmeldung erwahnt
- Methods in Enzymology, Band 155, 1987, Academic Press, Inc., (Orlando, FLA, US), r.M. MYERS et al.: "Detection and Localization of Single base Changes by Denaturing Gradient Gel Electrophoresis", seiten 501-527 sieh seite 502, zeilen 22-33; seite 505, zeile 40 - seite 506, zeile 20
- Nucleic Acids Research, Band 13, Nr. 9, 10. Mai 1985, IRL, (Oxford, GB), R.M. MYERS et al.: "Nearly all Single base Subsitutions in DNA Fragments joined to a GC-Clamp can be Detected by Denaturing Gradient Gel Electrophoresis", seiten 3131-3145 siehe Zusammenfassung; seite 3132, zeilen 10-35; seite 3133, zeilen 4-15
- Proceedings of the National Academy of Sciences USA, Band 86, Januar 1989, V.C. SHEFFIELD et al.: "Attachment of a 40-base-pair G+C-rich Sequence (GC-Clamp) to Genomic DNA Fragments by the Polymerase chain Reaction Results in Improved Detection of Single-base Changes", seiten 232-236 siehe Zusammenfassung; seite 232, spalte 2, zeilen 23-43 in der anmeldung erwahnt
- Nucleic Acids Research, Band 16, Nr. 23, 23. Dezember 1988, IRL, (Oxford, GB), A.-C. SYVANEN et al.: "Quantification of Polymerase chain Reaction Products by Affinity-based Hybrid Collection", seiten 11327-11338 siehe Zusammenfassung
- Nucleic Acids Research, Band 18, Nr. 9, 11. Mai 1990, IRL, (Oxford, GB), R.M. WARTELL et al.: "Detecting base pair Substitutions in DNA Fragments by Temperaturegradient Gel Electrophoresis", seiten 2699-2705 siehe den ganzen artikel; Insbesondere die Zusammenfassung

## Beschreibung

Gegenstand der vorliegenden Erfindung ist ein Verfahren zur Trennung und Detektion von Komponenten eines Stoffgemisches durch Temperaturgradienten-Gelelektrophorese (TGGE), insbesondere ein Verfahren zur Detektion von Mutationen von Nukleinsäurefragmenten durch Analyse des durch Hybridisierung des die Mutation aufweisenden Nukleinsäurefragments (Mutante) mit dem diese Mutation nicht aufweisenden Nukleinsäurefragments (Wildtyp) gewonnenen Heteroduplexes, ein Verfahren zur Probenaufbereitung unter Verwendung eines Oligonukleotids sowie dieses Oligonukleotid selbst, sowie eine Vorrichtung zur Durchführung der Temperaturgradienten-Gelelektrophorese.

Der Nachweis von Mutationen in genetischem Material oder der Nachweis der phänotypischen Konsequenz einer genetischen Mutation ist eine wichtige analytische Aufgabe in vielen Bereichen biologischer Forschung, angewandter Medizin, biotechnischer Produktion und Kriminalistik. Auf genetischer Ebene bedeutet eine Mutation den Austausch mindestens eines Nukleotides oder Basenpaares auf DNA- oder RNA-Ebene. Die Möglichkeiten des sogenannten "genetic engineering" erlauben mit Hybridisierungs- oder Sequenzierungstechniken den Nachweis einer Mutation in klonaler DNA oder RNA. Diese Techniken beschränken sich jedoch auf forschungsnahe Anwendungen. Für den Routine-einsatz konnte ein technischer Standard, der dem Vergleich mit immunologischen Methoden (ELISA usw.) standhält, nicht erreicht werden.

Die Temperaturgradienten-Gelelektrophorese (TGGE), wie sie in der DE-OS 36 22 591 beschrieben wurde, ist eine Methode zur Detektion geringer struktureller Unterschiede oder Besonderheiten biologischer Makromoleküle wie Nukleinsäuren oder Proteine. Die Technik ist jedoch ungeeignet für eine automatisierbare Analyse, wie sie zum Beispiel bei Bestimmung vieler Einzelproben im klinischen Bereich bei der Analyse genetischer Erkrankungen oder in der forensischen Analytik notwendig ist. Mit Hilfe der TGGE-Technologie gelang es, Mutationen ohne umständliche differentielle Hybridisierung direkt sichtbar zu machen (Riesner et al. (1989), Electrophoresis 10, S. 377-389), jedoch beschränkt sich die Technik auf die Handhabung einer Flachbett-Gelelektrophorese für die forschungsmäßige Analytik. Ähnliche Resultate liefert die Kombination aus Flachbett-Gelelektrophorese und einem denaturierenden chemischen Gradienten, der sich aber kaum reproduzierbar formen läßt und somit für eine Automatisierung nicht in Frage kommt.

Die notwendige Voraussetzung für die empfindliche Erfassung selbst einzelner Mutationen in der TGGE ist ein homogenes Temperaturniveau im Gel senkrecht zur Laufrichtung der Elektrophorese, d.h. an Orten gleichen elektrischen Potentials. Dies kann zum Beispiel durch die doppelseitig temperierte senkrechte Elektrophorese nach D.R. Thatcher und B. Hodson (1981), Biochemistry 197, S. 105-109, nicht hinreichend realisiert werden, da die thermisch nicht ausreichend verbundenen gegenüberliegenden Thermostatierplatten an Orten gleichen elektrischen Potentials nicht identische Temperaturen aufweisen.

Das Erscheinungsbild der belebten Natur ist auf genetischer Ebene in Form der Nukleinsäuren programmiert, bestehend entweder aus RNA- oder DNA-Kettenmolekülen. Veränderungen der genetischen Information werden als Mutationen bezeichnet und sind die Basis für evolutionäre Entwicklungen, für genetisch bedingte Erkrankungen und andere genetisch bedingte biologische Eigenschaften eines Virus oder Organismus. Die Mehrzahl der erfolgten Mutationen ist für das System ohne erkennbare Auswirkung. Solche Mutationen werden als neutral bezeichnet. Seit Einführung gentechnologischer Techniken ist es möglich geworden, eine Mutation zu entdecken, ihre zeitliche Entstehung zu bestimmen und ihren Einfluß auf eine biologische Funktion zu messen.

Mit der Methode der vergleichenden Sequenzanalyse (Sequenzierung) von homologen Sequenzen wird eine Mutation erkennbar. Unter dem Begriff Mutation wird ein einzelner Nukleotidaustausch, eine Deletion oder eine Insertion einzelner bis vieler Nukleotide oder eine Umordnung von Kettensegmenten verstanden. Die Sequenzanalyse ist trotz großer Fortschritte in den letzten Jahren nach wie vor eine aufwendige Technik, unterstützt von kostspieligen Apparaturen und für Reihenanalysen ungeeignet. Lediglich die Suche nach an sich bekannten Mutationen, wie sie für bestimmte Erberkrankungen, zum Beispiel alpha-1-Antitrypsin-Defizienz (Kidd, U.J., Wallace, R.B., Hakura, K. u. Woo, S.L.C. (1983), Nature 304, 230-234) beschrieben sind, hat sich technisch durch die Verwendung synthetischer Oligonukleotidsonden vereinfacht. Eine Anzahl von Fragestellungen entzieht sich jedoch nahezu vollständig dem experimentellen Zugriff, wie zum Beispiel die Suche nach unbekannten Mutationen auf langen Gensegmenten, die nicht mit Restriktionsfraqmentlänqenpolymorphismen (RFLP) verknüpft sind, oder Reihenuntersuchungen, die für die medizinische Genetik, Populationsanalysen, evolutionäre Verwandtschaftsanalysen, Virusvariantenanalysen usw. von Bedeutung sind.

Nukleinsäureketten (RNA und DNA) haben die Fähigkeit, mit sogenannten Komplementärsequenzen Doppelstrangstrukturen auszubilden. Es entstehen dabei DNA/DNA, RNA/RNA und DNA/RNA Doppelstrangstrukturen. Eine charakteristische Eigenschaft dieser Strukturen ist ein temperaturabhängiges Denaturieren (Schmelzen) der Doppelstränge. Das Schmelzen geschieht in einem sehr engen Temperaturintervall, d.h. es denaturieren große Abschnitte der Doppelstrangstruktur in einem einstufigen Prozeß. Es handelt sich somit um hochkooperativ ablaufende physikalische Reaktionen. Der Verlust der durchgängigen Doppelstrangstruktur äußert sich in einer veränderten Mobilität (meist ein Mobilitätsverlust) der betroffenen Nukleinsäure. Dieser Mobilitätsverlust kann in einem elektrophoretischen Trennverfahren ausgenutzt werden, um Nukleinsäuren verschiedener Schmelztemperaturen zu trennen. So wandern thermodynamisch instabilere Nukleinsäuren langsamer und somit weniger weit als solche mit stabilen Strukturen. Das zur Trennung verwendete Medium muß dabei einen Denaturierungsgradienten aufweisen, beispielsweise durch steigende Konzentration eines denaturierenden Agens. Die Stabilität der internen Bereiche der Basenpaare ist abhängig vom G/C-Gehalt sowie der Sequenz. Diese Effekte sind ausführlich untersucht worden (Meinkoth, S. u. Wahl, G. (1984), Analytical Biochem. 138, 267-284).

Führt die Mutation nun zu hinreichend großen Veränderungen in dem entsprechenden Bereich, so wird die mutierte Nukleinsäure ein anderes Schmelzverhalten aufweisen als die nicht mutierte. Häufig ist eine Mutation nur durch den Austausch eines Basenpaares gegen ein anderes Basenpaar gekennzeichnet (Transversion oder Transition). Daher ist der mutierte Nukleinsäurestrang in sich recht stabil und schmilzt in der Regel bei ähnlichen Temperaturen wie die nicht mutierte Form, so daß eine Diskriminierung nicht möglich ist. Solche Mutationen werden jedoch sichtbar, indem die mutierte Nukleinsäure mit einer Nukleinsäure, welche diese Mutation nicht aufweist (Wild-typ), in vergleichbaren Konzentrationen gemischt, bis einschließlich der Strangtrennung denaturiert und danach wieder renaturiert wird. Dadurch entstehen alle Kombinationen der entsprechenden Nukleinsäure-Einzelstränge, unter anderem auch sogenannte Heteroduplices aus mutiertem Einzelstrang und nicht mutiertem Einzelstrang. Da in den Heteroduplices nun dem jeweiligen Nukleotid an der Stelle der Mutation das komplementäre Nukleotid fehlt, kommt es an diesen Positionen zu merklichen Destabilisierungen in den benachbarten Doppelhelix-Regionen. Die Heteroduplices werden dementsprechend eher aufschmelzen als die Duplices des Wildtyps oder der Mutante.

Nuc.Ac.Res. 16/23, 1988, pp. 11327-11338 beschreibt die Kombination der polymerase chain reaction mit einer Hybridisierungsreaktion in Lösung, bei der die gebildeten Hybridsequenzen mittels einer biotinylierten Sonde an eine feste Matrix (z.B. avidin-beschichtete Polystyrolpartikel) gebunden werden. Die Hybridisierung der amplifizierten Sequenzen mit der radioaktiv markierten Sonde erfordert einen kombinierten De-/Renaturierungsschritt.

Nachteilig an den bislang bestehenden Verfahren sind die relativ umständliche Handhabung sowie die nicht immer sicher zu gewährleistende Aufspürung der vermuteten Mutationen.

Das der Erfindung zugrunde technische Problem ist somit einmal, ein Verfahren bereitzustellen, das die Nachteile der Undurchführbarkeit der TGGE für den analytischen Routinebetrieb beseitigt und die TGGE insgesamt leichter handhabbar macht. Weiterhin soll eine Vorrichtung bereitgestellt werden, die eine automatische Auswertung der TGGE ermöglicht. Die Vorrichtung soll auch die simultane Analyse von strukturell stark variierenden Nukleinsäuren ermöglichen. Ein weiteres technisches Problem ist die Verbesserung der Aufspürung, insbesondere die quantitative und/oder qualitative Erfassung, von Mutationen und/ oder Genvarianten. Die Verbesserung soll in einer bestimmten Ausführungsform auch eine einfachere und sicherere Probenvorbereitung gewährleisten.

Erfindungsgemäß werden diese technischen Probleme gelöst durch ein Verfahren zur quantitativen und qualitativen Detektion von Mutanten oder spezifischen Gensequenzen, wobei einem Gemisch differierender Sequenzen, deren eine Konzentration bekannt sein kann, im Unterschuß ein Marker-Nukleinsäuremolekül zugesetzt wird, dessen Sequenz mit der Sequenz einer der vorgenannten Sequenzen mit Ausnahme der Markierung identisch ist und dieses Gemisch mindestens einem Denaturierungs- und Renaturierungszyklus unterworfen und analysiert wird, wobei die nach einem Denaturierungs-/Renaturierungszyklus gebildeten Hybride mittels Temperaturgradientengelelektrophorese (TGGE) getrennt werden und deren relative Signal intensitäten gemessen werden.

Erfindungsgemäß kann die Detektion von Mutationen von Nukleinsäuren quantitativ und qualitativ durchgeführt werden, und zwar durch Analyse des durch Hybridisierung des die Mutation aufweisenden Nukleinsäurefragments (Mutante) mit dem diese Mutation nicht aufweisenden Nukleinsäurefragment (Wildtyp) gewonnenen Heteroduplexes, wobei das die Mutation tragende, zu untersuchende Nukleinsäurefragment so gewählt wird, daß die Mutation in einer thermodynamisch instabilen Region des Heteroduplexes liegt.

Der räumliche Temperaturgradient, der im erfindungsgemäßen Verfahren einsetzbar ist, kann so aufgebaut werden, daß mit einer regelbaren Heiz- oder Kühlvorrichtung auf der Probenseite ein bestimmtes Temperaturniveau eingestellt wird, während das zweite, räumlich getrennte Temperaturniveau durch die Temperatur des Elektrophoresebades auf der gegenpoligen Seite definiert ist. Diese einfache Verfahrensweise ist dann möglich, wenn das Elektrophoresebad so dimensioniert ist, daß die Temperatur konstant bleibt. Dazu ist ein hinreichend groß dimensioniertes Elektrophoresebad notwendig. Vorzugsweise wird jedoch auch hier das zweite Temperaturniveau etwa durch Peltierelemente, Heizdrähte oder thermostatierbare Wasserbäder regelbar ausgestaltet.

Nach dem Probenauftrag werden die Komponenten des zu trennenden Stoffgemisches längs des elektrischen Feldes in das Trennmedium hineinwandern. Dabei erreichen sie das erste Temperaturniveau und erfahren eine Konformationsumwandlung, die in einer drastischen Reduktion der Wanderungsgeschwindigkeit resultiert. Dies wird entweder direkt durch die eingestellte Temperatur erreicht oder in Kombination mit partiell denaturierenden Reagenzien. Sind die zu trennenden Komponenten beispielsweise Nukleinsäuren, werden durch die partielle Denaturierung die Doppelstränge teilweise zu größeren "Loops" aufgeweitet, die in dem Trennmedium quasi steckenbleiben und der Elektrophorese nicht mehr folgen können. Wird nun die Temperatur gesenkt, so bilden sich die "Loop"-Regionen in Abhängigkeit ihrer thermodynamischen Stabilität zurück, wodurch die Mobilität der Nukleinsäuren in dem Trennmedium wieder erhöht wird. Die jeweilige Temperatur ist eine Stoffcharakteristik der entsprechenden Nukleinsäure. Somit gelingt es, die verschiedenen Komponenten in Abhängigkeit der Temperatur zu trennen. Die Moleküle, deren Mobilität erhöht wurde, wandern durch die Trennstrecke und können am Zielpol der Elektrophorese detektiert werden.

Es ist ebenfalls möglich, einen Temperaturgradienten mit steigender Temperatur in Elektrophorese-Richtung auszubilden, in welchem die Moleküle zunächst nach ihrem thermodynamisch partiellen Aufschmelzverhalten in dem Trennmedium getrennt werden. Bei tieferer Temperatur werden zunächst die thermodynamisch instabilsten Strukturen partiell thermisch denaturieren, zum Beispiel bei Nukleinsäuren durch Ausbildung von "Loop"-Regionen. Die Mobilität dieser Nukleinsäuren wird drastisch gesenkt, so daß diese in dem Trennmedium entweder "steckenbleiben" oder zumindest nur noch sehr langsam wandern. Die übrigen Komponenten wandern weiter durch das Trennmedium, bis jeweils ihre spezifische Denaturierung zum drastischen Mobilitätsverlust der jeweiligen Moleküle in dem Trennmedium führt. Bei bestimmter Wahl der Maschengröße des Trenngels führt die Restmobilität der quasi arretierten Biomoleküle dazu, daß nach einiger Zeit - wenn auch sehr langer Zeit - alle Komponenten des zu trennenden Gemisches die Trennstrecke durchwandern können. Unterstützt wird dieser Effekt dadurch, daß die Mobilität der partiell denaturierten Nukleinsäuren bei vollständiger Denaturierung - also Trennung in die Einzel stränge - wieder drastisch zunimmt, da jetzt nur noch die Einzelstränge durch das Trennmedium wandern. Die thermodynamisch instabilsten Nukleinsäuren, die bei tiefen Temperaturen bereits ihre Mobilität verlieren, wandern zwar sehr langsam im Gel weiter, erreichen jedoch irgendwann ein höheres Temperaturniveau, das zum vollständigen Aufschmelzen führt, so daß die Doppelstränge in ihre Einzelstränge zerfallen. Dies führt dann zu der oben beschriebenen beschleunigten Wanderungsgeschwindigkeit.

Dieser Effekt kann auch zur Beschleunigung der Elektrophorese insgesamt ausgenutzt werden. Sind die Komponenten durch Teildenaturierung und Mobilitätsverlust in dem Trennmedium voneinander getrennt worden, dann kann das Temperaturniveau des gesamten Trennmediums über den Schmelzpunkt der Doppelstränge hinaus erhöht werden, um alle Doppelstränge vollständig in Einzelstränge zu überführen. Danach gewinnen alle Komponenten ihre Mobilität zurück. Zur Durchführung dieser Verfahrensweise ist es jedoch erforderlich, daß die Temperatur entweder sehr schnell über das gesamte Trennmedium äquilibriert wird oder die Elektrophorese bis zur Äquilibrierung der Temperatur unterbrochen wird, beispielsweise durch Abschaltung des elektrischen Feldes. Vorzugsweise sind die zu trennenden Moleküle von ähnlicher Größe. Die Verfahrensweise unter den beschriebenen Bedingungen gewährleistet dann bei der isothermischen Elektrophorese mit hohem Temperaturniveau, daß die getrennten Komponenten im weiteren Verlauf der Elektrophorese ihren relativen räumlichen Abstand beibehalten.

Eine andere Verfahrensweise benutzt lediglich ein zeitlich gesteuertes variables Temperaturprogramm vorzugsweise an der Probenauftragsseite der Elektrophorese zum Aufbau eines zeitlichen Temperaturgradienten, der zur Trennung der Komponenten des Stoffgemisches führt. Man läßt die zu trennende Probe zunächst elektrophoretisch in das Trennmedium hineinwandern. Das Temperaturniveau auf der Probenauftragsseite ist so gewählt, daß beispielsweise bei Nukleinsäuren die Doppelstränge zu "Loops" aufgeweitet sind, ohne jedoch vollständig aufgeschmolzen zu werden. Die Bildung von "Loops" kann durch geeignete Reagenzienwahl unterstützt werden. Dies führt dazu, daß die zu analysierenden Komponenten am Beginn der Elektrophorese in dem Trennmedium quasi arretiert sind. Wird nun die Temperatur abschnittsweise gesenkt, so bilden sich die thermodynamisch stabilsten Doppel stränge zurück, so daß diese Nukleinsäuren eine erhöhte Mobilität aufweisen. Diese beginnen dann durch das Trenngel zu wandern. Durch die nachfolgende Temperaturänderung werden dann nachfolgend die Nukleinsäuren mit der nächstniedrigeren thermodynamischen Stabilität mit der Wanderung beginnen. Es kann vorteilhaft sein, die Temperaturabsenkung schrittweise durchzuführen, um den Trennungseffekt in räumlicher Hinsicht zu verstärken. Die mit der Wanderung beginnenden Moleküle gewinnen quasi einen räumlichen Vorsprung beim Durchwandern des Trennmediums. Bei hinreichend großem Abstand der thermodynamischen Stabilität ist es jedoch auch möglich, den Temperaturgradienten relativ schnell kontinuierlich zu senken. Bei dieser Verfahrensweise kann es vorteilhaft sein, auch den Elektrophorese-Endpunkt mit einer regelbaren Heiz- oder Kühlvorrichtung zu versehen.

Bei allen erfindungsgemäßen Verfahrensweisen ist es jedoch notwendig, zum Aufbau eines reproduzierbaren Temperaturgradienten bzw. reproduzierbarer isothermischer Bedingungen das Trennmedium mit einem thermisch leitenden Thermostatiermantel zu umschließen. Zur Ausbildung des reproduzierbaren Temperaturgradienten bzw. des isothermen Temperaturniveaus ist es unbedingt erforderlich, daß der Energiefluß im Thermostatiermantel klein ist gegenüber den Energieflüssen in Heiz- und Kühlelement.

Die zur Temperatur-Gelelektrophorese verwendbaren Temperaturen liegen vorzugsweise im Bereich von 0 bis 100°C. Das Trennmedium besteht vorzugsweise aus Polyacrylamid-Gelen.

Das technische Problem der Detektion von Komponenten eines Stoffgemisches, bei dem das Gemisch aus Nukleinsäuren besteht, die insbesondere lediglich eine Mutation aufweisen, wird durch ein Verfahren gemäß Patentanspruch 4 gelöst. Die Unteransprüche 5 bis 22 sind bevorzugte Ausführungsformen des erfindungsgemäßen Verfahrens. Die Ansprüche 23 bis 25 betreffen Verwendungen von Mitteln zur Durchführung des erfindungsgemäßen Verfahrens.

Das erfindungsgemäße Detektionsverfahren für Mutationen in Nukleinsäuregemischen wird im folgenden näher erläutert.

Das für die Mutationsanalyse in Frage kommende Nukleinsäurefragment wird zunächst auf eine passende Größe geschnitten. Dabei wird die Mutation in einen thermodynamisch labilen Bereich gelegt. Dieser Bereich kann entweder experimentell oder auch durch Berechnung ermittelt werden. Gegebenenfalls kann das Nukleinsäurefragment durch PCR (polymerase chain reaction) amplifiziert werden. Dabei wird vorzugsweise die thermodynamisch stabilere Region so stabilisiert, daß sie unter maximal denaturierenden Bedingungen des analytischen Experimentes noch stabil bleibt. Das Nukleinsäurefragment schmilzt dann nicht bis zur vollständigen Strangtrennung auf, sondern bildet eine Y-förmige Struktur geringer elektrophoretischer Mobilität aus. Insbesondere kann diese Stabilisierung durch Anfügung einer Region aus stabilisierenden G/C-Nukleotiden oder ungeladenen Nukleotiden herbeigeführt werden. Der stabilisierende Einfluß von mehr als 40 G/C-Basenpaaren wurde bereits von Sheffield, V.C. et al. (1989) Proc. Natl. Acad. Sci. USA 86, 232-236, beschrieben. Überraschenderweise hat sich jedoch gezeigt, daß bereits 20 bis 30 G/C-Basenpaare zur Stabilisierung ausreichend sind. Vorzugsweise bedient man sich der Temperaturgradienten-Gelelektrophorese, wie sie erfindungsgemäß beschrieben wird. Aber auch die Methode der DE-OS 36 22 591 kann Verwendung finden, wobei der Temperaturgradient in einer Plattenelektrophoresevorrichtung ausgebildet wird und die Trennung mit einem auf einer Platte angeordneten Gel durchgeführt wird.

Will man bestimmte Nukleinsäurefragmente durch Amplifizierungsreaktionen wie der sogenannten PCR (polymerase chain reaction) vervielfältigen, werden dazu Oligonukleotide als sogenannte Primer verwendet. Diese Primer werden so gewählt, daß sie in der Lage sind, mit einem Teil der zu untersuchenden Nukleinsäure zu hybridisieren. Vorteilhafterweise sollen die Sonden mit den endständigen Regionen dieser zu untersuchenden Nukleinsäuren hybridisieren. Des weiteren ist es wünschenswert, die Primer mit Restriktionsschnittstellen zu versehen, um die amplifizierten Segmente in Vektoren einbauen zu können. In einer weiteren bevorzugten Ausführungsform weisen die Primer endständig chemische Gruppen auf, die als Affinitätsliganden dienen können.

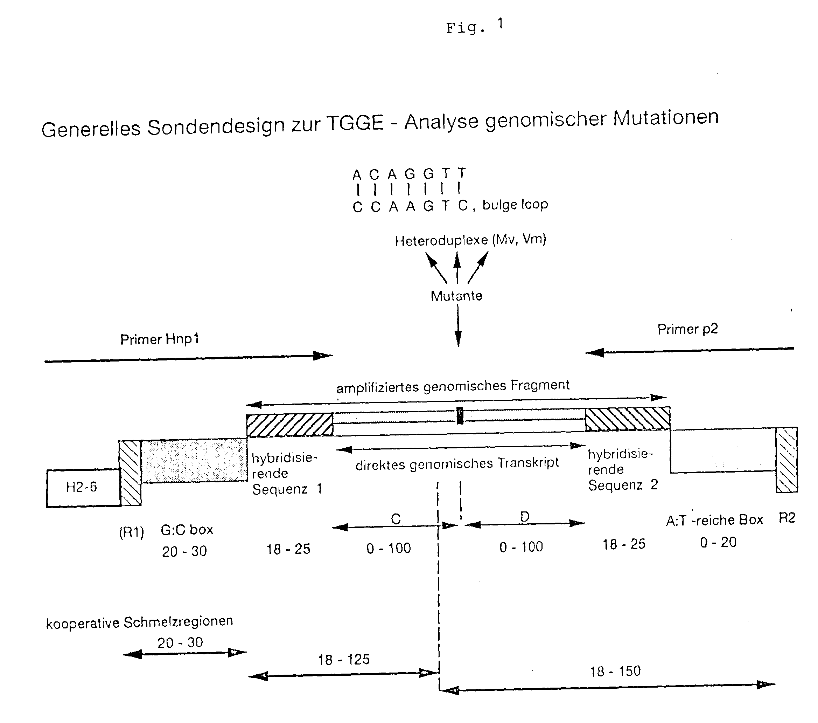

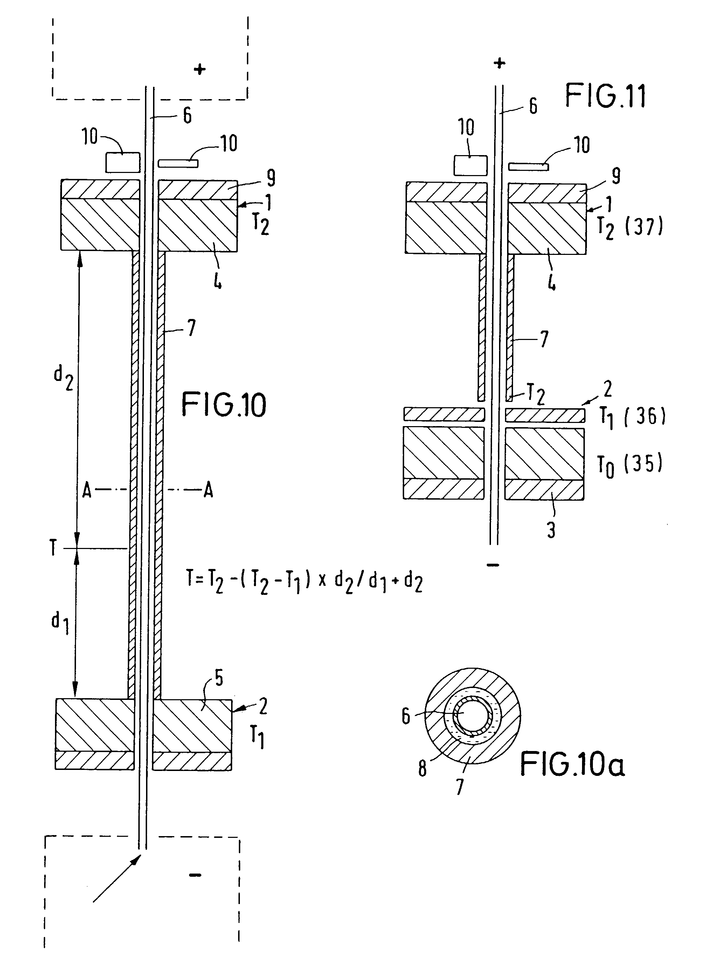

In Figur 1 ist ein besonders bevorzugtes Oligonukleotid schematisch dargestellt, welches als Primer in einer PCR Verwendung finden kann. Der Primer Hnpl besteht aus einer hybridisierenden Sequenz 1, welche etwa 18 bis 25 Nukleotide aufweist. Zum freien Ende schließt sich eine G/C-Box aus vorzugsweise 20 bis 30 Nukleotiden an, gefolgt von einer Restriktionsschnittstelle sowie einer oder mehreren chemischen Gruppen, die als Affinitätsliganden dienen können. Der Primer p2 besteht ebenfalls aus einer hybridisierenden Sequenz 2, einer A/T-reichen Box mit 0 bis 20 Nukleotiden Länge und einer Restriktionsschnittstelle R2. Im Falle der Mutantenanalyse innerhalb hochpolymorpher Regionen ist es empfehlenswert, die Größe der Regionen C, D (Fig. 10) so klein wie möglich, im Extremfall auf 0 Nukleotide zu halten, um nur eine polymorphe Position zu erfassen. Dies sei an einem Beispiel der Sondenkonstruktion zum Nachweis der β-Globin Thalassämie "Yugo" (IVS-1-6, T-C) exemplarisch ausgeführt.

Ein wichtiger Typ einer β-Globin Thalassämie ist die Splicing-Mutante (IVS-1-6, T-C) auf dem β-Globin Locus.

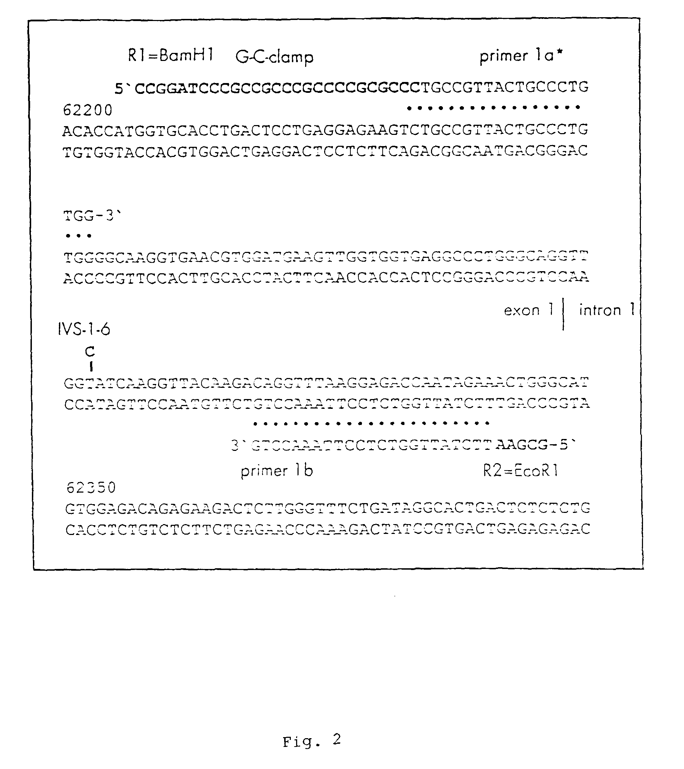

Diese Mutation verhindert das korrekte Splicing zwischen Exon 1 und Exon 2. Die Umgebung des mutierten Genortes IVS-1-6 ist in Figur 2 wiedergegeben. Es wird ein Ausschnitt des humanen βgb-Locus 62200 bis 62350 (GenBank-Sequenz HUM HBB_PREMRNA) mit intron-Mutante IVS-1-6 und den erfindungsgemäß verwendeten Primern gezeigt.

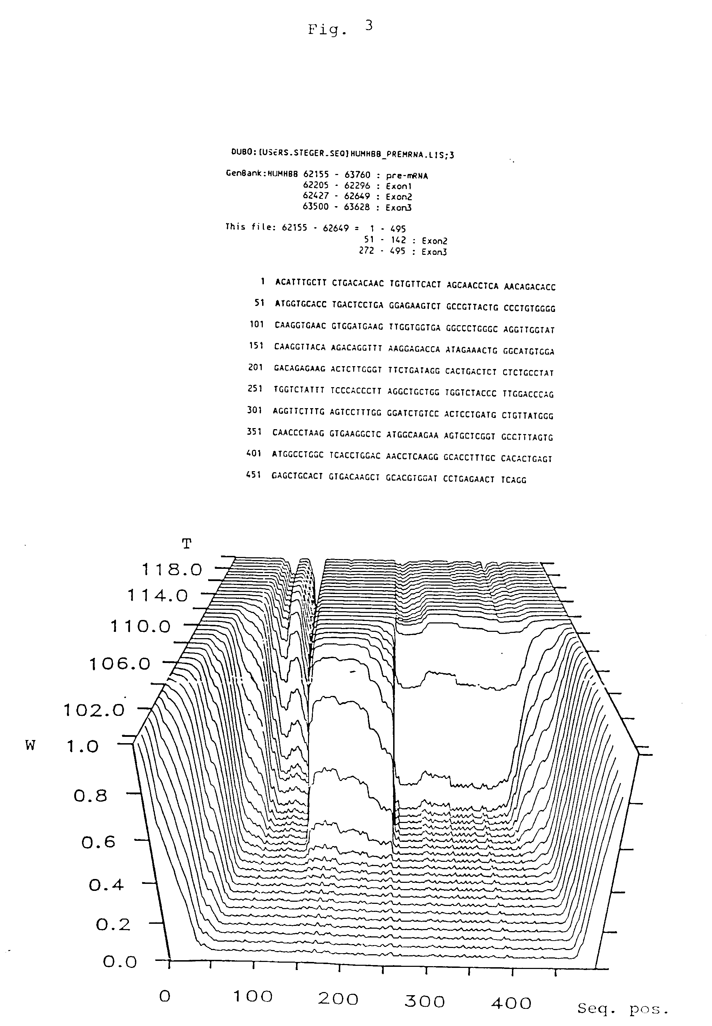

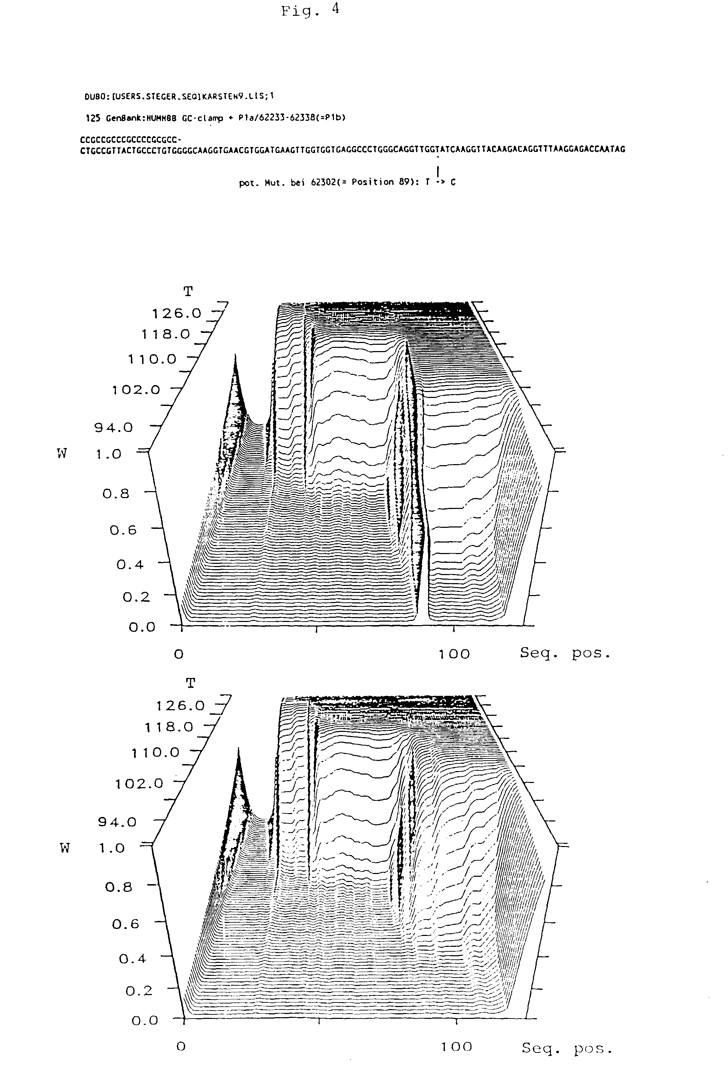

Figur 3 zeigt das berechnete Schmelzverhalten dieser Nukleinsäure, erhalten unter Anwendung des von Gerhard Steger et al. entwickelten Rechenschemas (Steger, G., Po, T., Kaper, J. u. Riesner, D. (1987), Nucleic Acids Res. 15, 5085-5103). Das in Figur 2 wiedergegebene Segment schließt die intron 1-β⁺-Mutation IVS-1-6 ein. Die Numerierung korrespondiert mit der GenBank Sequenz HUM HBB_PREMRNA. Die optimale Sonde für denaturierende Gele basiert auf dem amplifizierten Segment 62233-62340. Der Primer 1a* ist mit einem sogenannten G/C-Schwanz konstruiert. BamH1- und EcoR1-Restriktionsschnittstellen wurden zur Integration in den Vektor pBR322 gewählt. Die Berechnungen für die verschiedenen DNA-Denaturierungszustände von β-Globin-Segmenten bei verschiedenen Temperaturen für 1 M NaCl sind graphisch dargestellt. Das in Figur 3 dargestellte Schmelzdiagramm ist wie folgt zu verstehen: Auf der Abszisse ist die Position der Nukleinsäuren angegeben, während die Ordinate die Wahrscheinlichkeit der Strangöffnung repräsentiert, bezogen auf die spezifische Nukleotidposition. Die dritte Achse des dreidimensionalen Diagramms entspricht einer Temperaturachse, wobei zu beachten ist, daß die Schmelztemperaturen ebenfalls abhängig sind von der Ionenstärke des Mediums. Die Wahrscheinlichkeit für den geöffneten Zustand eines Basenpaares ist in Schritten von 0,5°C berechnet. Die dreidimensionale Zeichnung illustriert das sequentielle Denaturierungsverhalten verschiedener kooperativ denaturierender Regionen. Wie in Figur 4 dargestellt, erniedrigt ein interner Loop von der Größe eines Basenpaares bei Position 62302 die Schmelztemperatur der Region mit der geringsten Stabilität.

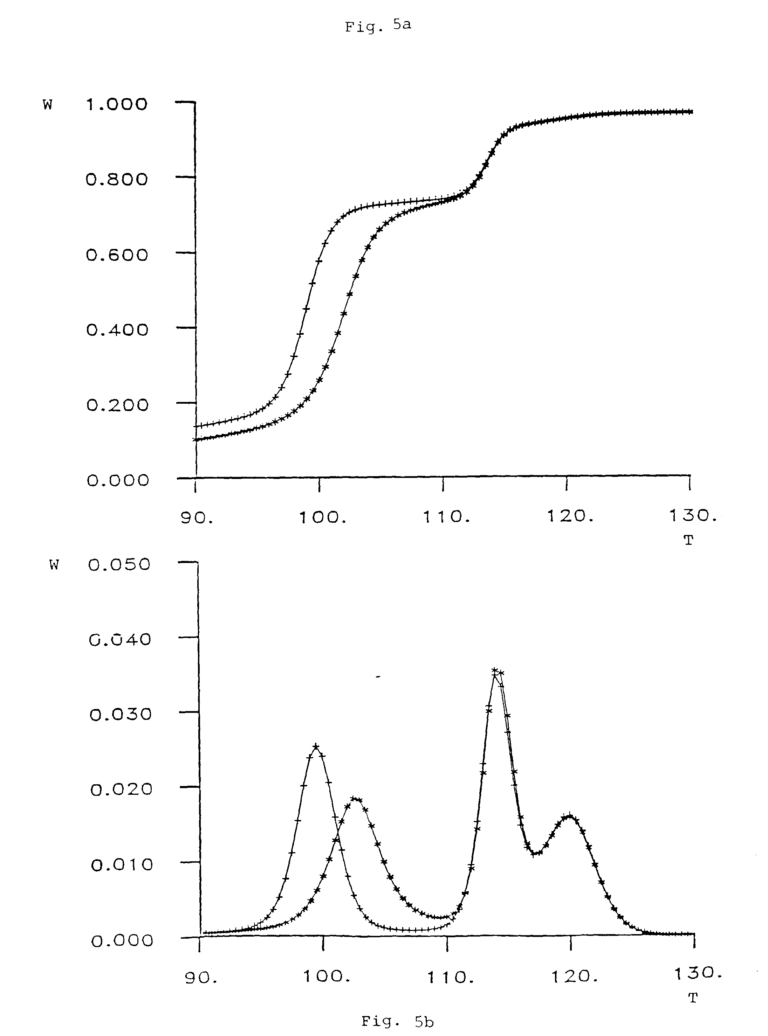

Die Figur 5 betrifft die Berechnung der temperaturabhängigen Schmelzkurve in integrierter Form (5a) und in der differentiellen Form (5b), wobei das Symbol (*) den Kurvenverlauf des Wildtyp-Homoduplex und (+) den Kurvenverlauf des Heteroduplex (Mv) als A/C-Fehlpaarung bedeuten.

Die Figur 5a zeigt die theoretisch abgeleitete optische Schmelzkurve des Nukleinsäuredoppelstrangs sowohl des Homo- als auch des Heteroduplexes (A/C). Figur 5b zeigt die erste Ableitung der in Figur 5a berechneten Schmelzkurve. Es ist erkennbar, daß der Heteroduplex (+++) im Bereich der thermodynamisch labilen Region infolge der Mutation deutlich stärker destabilisiert ist als der Homoduplex (***) aufgrund des in sogenannten Mismatches hervorgehobenen internen Loops. In diesem Fall beträgt die Schmelzpunkterniedrigung dieser thermodynamisch instabilen Region etwa 4°C gegenüber dem Homoduplex.

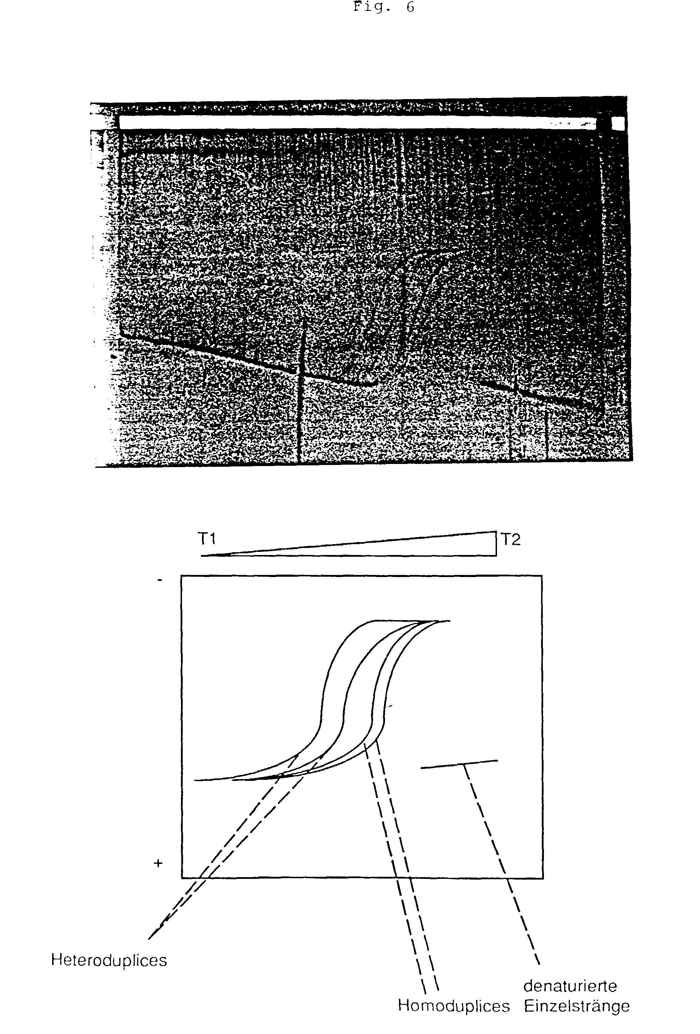

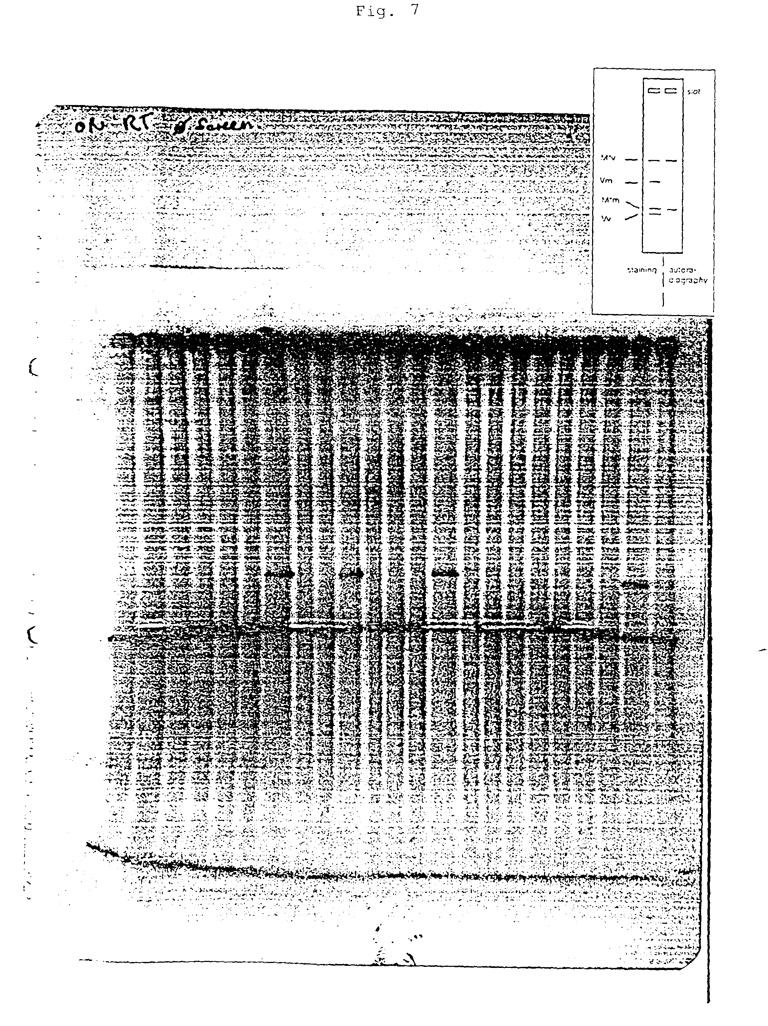

Die Figur 6 zeigt die Analyse des amplifizierten Mutanten-Segments mit Primer 1a*, Primer 1b gemäß Figur 2 und des amplifizierten Wildtyp-Segments auf der senkrechten Temperaturgradienten-Gelelektrophorese mit einem linearen Gradienten von 10 bis 60°C. Die äquimolar gemischten Fragmente wurden nach Denaturierung/Renaturierung aufgetragen. Die Heteroduplices (Mv, Vm) und die Homoduplices (Vv, Mm) werden aufgespalten gemäß der schematischen Darstellung. Eine homozygote DNA-Sonde vom Wildtyp wurde PCR-amplifiziert unter Einsatz der Primer 1a* wie oben beschrieben. Das Fragment wurde zwischen die BamHl-/EcoRl-Stelle von pBR322 integriert. Eine DNA-Probe der homozygoten Mutante IVS-1-6 wurde amplifiziert mit BamH1 und EcoRl geschnitten, denaturiert und hybridisiert mit einem klonierten BamH1-/EcoR1-Fragment der Wildtyp gb-Sequenz. Die resultierenden Homoduplices (2 Banden) und Heteroduplices (2 Banden) sind als vier unterschiedliche Banden repräsentiert. Die Y-förmigen Konformationen wurden stabilisiert durch den Einsatz der G/C-reichen Oligonukleotid-Kette am stabilsten Ende der zu untersuchenden Nukleinsäure (s. Myers, R.M. et al. (1985), Nucleic Acids Res. 13, 3131). Bei Anwendung der parallelen Temperaturgradienten-Gelelektrophorese ergibt sich bei Mutation IVS-1-6 im Probenmaterial das in Figur 7 dargestellte Bild.

Die Figur 7 zeigt die Autoradiographie einer Analyse verschiedener Patienten-DNAs nach Amplifikation und Test mit dem Wild-Typ Standard (s. Fig. 6, Fig. 2). Die Patienten-DNAs, die die IVS-1-6-Mutation enthalten, weisen eine zweite Bande auf. Da die Test-DNA nur einen markierten Strang enthält, enthalten nur zwei der vier Banden die radioaktive Markierung (s. Einschub in Fig. 7). Der parallele Temperaturgradient verläuft zwischen 25 und 65°C.

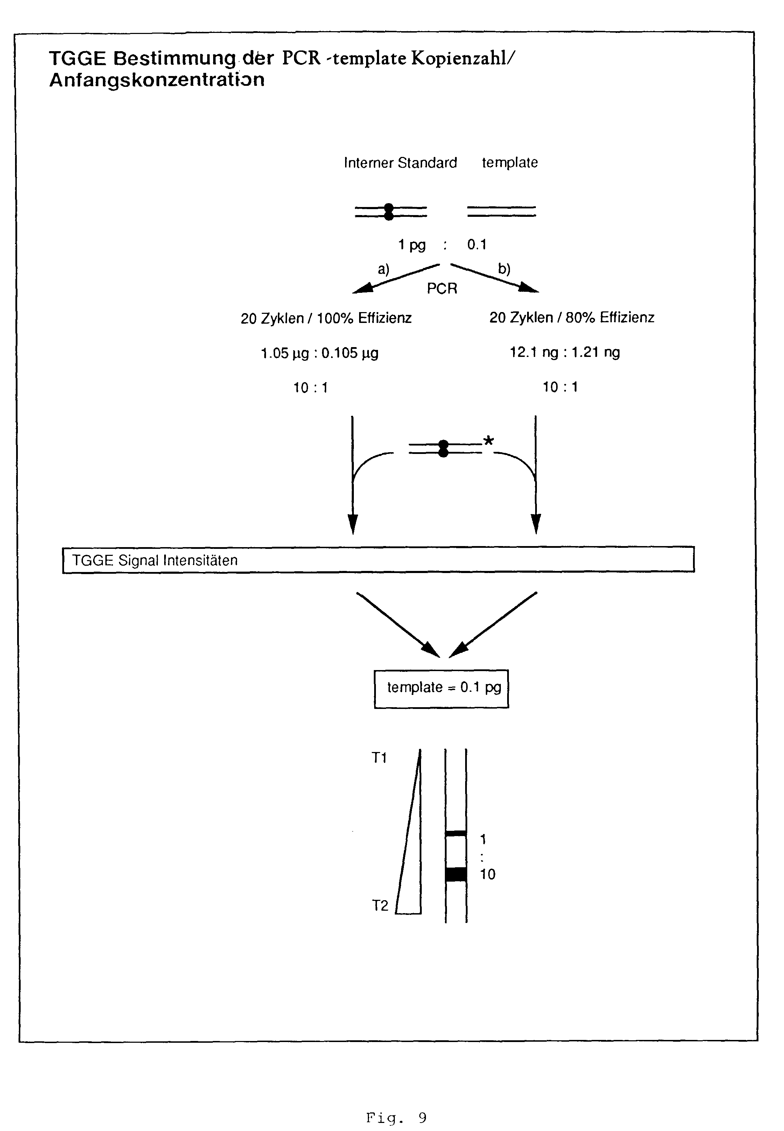

Das Experiment zeigt darüberhinaus, daß das Mutantennachweissystem nicht nur zum qualitativen sondern auch zum quantitativen Nachweis geeignet ist. Das im molaren Unterschuß zugesetzte (radioaktiv) markierte Markerfragment (Wildtyp) verteilt sich nach Denaturierung/Renaturierung anteilig auf die beiden Allele. Die Mutation des einen Allels hat keinen Einfluß auf die 1:1 Verteilung des Markers (Fig. 7). Der Marker selbst trägt nur unterhalb der experimentellen Fehlergrenze (± 10 %) zur Verschiebung des 1:1 Verhältnisses bei. Wenn der interne Standard wie im Fall der gleichverteilten Allele nicht im zu amplifizierenden Gemisch vorhanden ist, kann er extern zugesetzt werden. Ein interner Standard definierter Kopienzahl kann dazu dienen, ein Template quantitativ zu erfassen, wenn es sich in einer Mutation vom Standard unterscheidet. Da exakt identische Primer eingesetzt werden, wirken sich Plateau-Effekte, ungleiche Primerkonzentrationen oder schlechte Replikationseffizienz immer streng symmetrisch auf beide Komponenten aus. Wenn sich Standard und zu messende Zielsequenz nicht um mehr als Faktor 100, vorzugsweise 10 unterscheiden, läßt sich die Kopienzahl über das Signalverhältnis korrekt ermitteln (Fig. 9). Experimentell bedeutet dieser Ansatz eine starke Vereinfachung, da die Polymerase Chane Reaction (PCR) unkontrolliert bis in die Sättigung getrieben werden kann.

Die Figur 9 verdeutlicht schematisch das Verfahren, das in Figur 7 beschrieben wird. Es wird der zu amplifizierenden DNA ein Standard bekannter Konzentrationen (Kopienzahl) zugesetzt, wobei sich der Standard in mindestens einer Mutation, z.B. einer Punktmutation, von der zu analysierenden Nukleinsäure unterscheidet. Diese Mischung wird einem enzymatischen Amplifizierungsverfahren bis zur Sättigung unterworfen. Danach wird dem Amplifikationsgemisch im Unterschuß der Standard in markierter Form zugesetzt. Nach mindestens einem Denaturierungs/Renaturierungszyklus wird die Markierung im zahlenmäßigen Verhältnis vom internen Standard und zu quantifizierender Zielsequenz in die entsprechenden Homo- und Heteroduplexe überführt. Die Trennung der Homo- und Heteroduplexe erfolgt mittels TGGE. Das Verhältnis der Signalintensität der entstandenen Banden ergibt nach Multiplikation mit der Standardkopienzahl die Menge der zu bestimmenden Zielsequenz.

Eine besonders einfache und effektive Probenvorbereitung für die Analyse der zu untersuchenden Nukleinsäure wird durch die Verwendung eines Primers mit Affinitätsgruppen ermöglicht. Als Affinitätsgruppen kommen beispielsweise Histidyl- und Biotinylreste in Frage. Im Fall der Histidylreste kommen zwei bis acht, besonders bevorzugt sechs Histidylreste zum Einsatz. Dieser chemisch modifizierte Primer wird dann an einen polymeren Träger über entsprechende Affinitätsgruppen fixiert. Im Fall der histidylmodifizierten Primer empfiehlt sich ein an einen polymeren Träger gebundener Chelatkomplex aus zweiwertigen Übergangsmetallionen, wie Kupfer und Nickel, und Nitrilotriessigsäure. Die freien Koordinationsstellen des Übergangsmetallions werden durch zwei Histidylreste besetzt. Da Primer und Histidylreste kovalent miteinander verbunden sind, wird auf diese Weise der Primer an die polymere Matrix gebunden. Komplexe sind für rekombinante Proteine (EP-A-0 282 042, EP-A-0 186 069) an NTA-Harze beschrieben worden (EP-A-0 253 303). Werden beispielsweise Biotinylreste kovalent an den Primer gebunden, empfiehlt sich ein polymerer Träger, der Avidinmoleküle kovalent gebunden hat.

Als polymere Träger kommen entsprechend modifizierte Membranen oder entsprechend modifizierte Partikel in Frage. Der polymere Träger soll mechanisch hinreichend stabil sein, um bei Operationen auftretende Druckschwankungen aufgrund von Durchfluß unbeschadet zu überstehen. Werden nun Nukleinsäuren mit Mutationen unter Verwendung des beschriebenen Primers amplifiziert, so bilden sich Doppelstränge aus, welche an einem Ende (Primer-vermittelt) besagte Affinitätsgruppen tragen. Nach erfolgter hinreichender Amplifizierung bringt man das Reaktionsgemisch mit dem polymeren Träger und daran gebundenen Affinitätsgruppierungen wie Nickelchelaten oder Avidinmolekülen zur Umsetzung. So werden spezifisch die durch den Primer amplifizierten Sequenzen an der Oberfläche des festen Trägers gebunden. Dies kann entweder in einem Batch-Verfahren oder in Form einer Säulenfiltration erfolgen. Durch diese Vorgehensweise werden die unerwünschten Enzyme und Reagenzien, welche zur Amplifikation benötigt werden, einfach und schonend abgetrennt. Die an der Matrix gebundenen amplifizierten Nukleinsäurefragmente können nun in einfacher Weise mit einer markierten Nukleinsäuresonde, die vom Wildtyp abgeleitet ist, inkubiert werden. Durch einen oder mehrere Denaturierungs-/ Renaturierungszyklen bilden sich Heteroduplexe, die nach Elution vom polymeren Träger, etwa durch Variation der Pufferbedingungen oder Auswaschen mit einem Kompetitor, der Analyse direkt unterzogen werden können. Die so aufgearbeiteten Proben können zum Beispiel unmittelbar der Temperatur-Gelelektrophorese unterzogen werden.

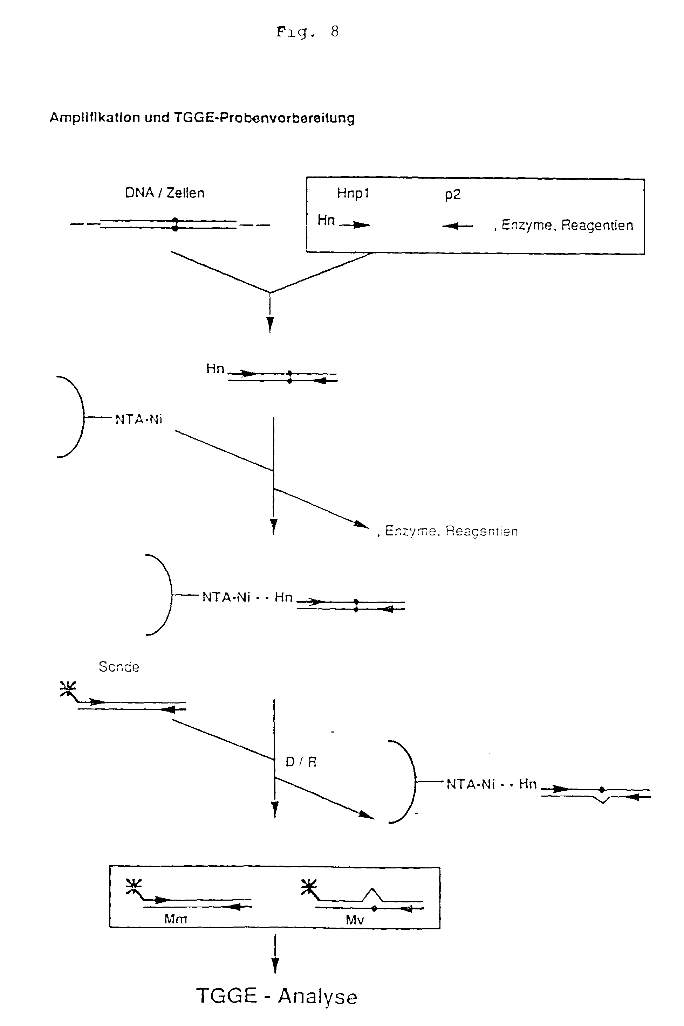

Die Figur 8 stellt ein erfindungsgemäßes Schema zur TGGE-gerechten Probenvorbereitung dar. Der Primer Hnpl trägt einen Oligo-Histidyl-Rest als 5'-gekoppelte Seitenkette und ist somit unter neutralen oder alkalischen Bedingungen an NTA-Liganden tragende Festphasenträger zu binden. Bei diesem Schritt werden kontaminierende Enzyme und Reagenzien entfernt. Nach Zugabe des Nachweisreagenzes und eventuell interner Markersubstanzen wird der Denaturierungs-/Renaturierungszyklus durchlaufen. Auf diese Weise werden die umrahmten Strukturen Mm und Mv (markierter Homoduplex/markierter Heteroduplex) zur TGGE-Analyse verfügbar. Das heißt, der zweite Strang des Analyten wird in der nachfolgenden TGGE-Analyse nachgewiesen.

Eine Vorrichtung zur Durchführung des erfindungsgemäßen Verfahrens besteht aus mindestens zwei Heiz- oder Kühlvorrichtungen oder einer Heiz- und einer Kühlvorrichtung zum Aufbau des Temperaturgradienten. Die Heiz- oder Kühlvorrichtungen sind mit Wärmereservoirs verbunden, um die nach dem erfindungsgemäßen Verfahren geforderten Energieflüsse zu gewährleisten. Die Wärmereservoirs und Heiz- oder Kühlvorrichtungen sind so ausgebildet, daß sie einen ein Trennmedium enthaltenden Hohlkörper vollkommen umgeben. Dieser Hohlkörper enthält die zur Trennung verwendete Trennmatrix oder das trägerfreie Trennmedium in seinem Lumen. Zum Aufbau des reproduzierbaren Temperaturgradienten oder bei isothermer Verfahrensweise zur Gewährleistung eines reproduzierbaren einheitlichen Temperaturniveaus des Trennmediums ist der Hohlkörper von einem Thermostatiermantel umgeben. Dabei kann der Thermostatiermantel vorzugsweise thermisch leitend mit dem Wärmereservoir oder den Heiz- oder Kühlvorrichtungen verbunden sein.

In einer bevorzugten Ausführungsform kann der Thermostatiermantel durch eine Metallplatte gebildet werden, die mit Bohrungen versehen ist, in die der das Trennmedium enthaltende Hohlkörper, vorzugsweise Glas- oder Kunststoffröhrchen, eingeführt werden können. Vorzugsweise werden zwei Metallplatten mit eingefrästen parallelverlaufenden Nuten verwendet, wobei die durch die Nuten entstehenden Aussparungen nach dem Zusammenlegen der Metallplatten der äußeren Form des zur Trennung verwendeten Hohlkörpers entsprechen und die Metallplatten in unmittelbarem Wärmekontakt stehen.

Die Figur 10 zeigt schematisch den Aufbau einer bevorzugten Ausführungsform der erfindungsgemäßen Vorrichtung. Figur la zeigt einen Querschnitt durch den von einem Thermostatiermantel umschlossenen Hohlkörper mit innen angeordnetem Trennmedium längs der Linie A --- A.

Die Figur 11 zeigt eine weitere bevorzugte Ausführungsform der erfindungsgemäßen Vorrichtung zum Aufbau eines zeitlichen Temperaturgradienten.

Die Figur 12 zeigt schematisch eine Ausführungsform der erfindungsgemäßen Vorrichtung, die eine Vielzahl der zur Trennung verwendeten Hohlkörper aufnehmen kann.

Die Figur 13 verdeutlicht schematisch den erfindungsgemäßen Verfahrensablauf mit einer Vorrichtung gemäß Figur 11.

Die Figur 14 und 14a zeigt eine bevorzugte Ausführungsform der erfindungsgemäßen Vorrichtung gemäß Fig. 12. Hierbei ist der Thermostatiermantel aus einer aus zwei Teilen bestehenden Metallplatte gebildet. Der röhrenförmige, das Trennmedium enthaltende Hohlkörper wird in auf beiden Platten parallelverlaufenden Nuten gehalten.

Die Figuren 14a, 15a und b zeigen einen seitlichen Querschnitt durch die Vorrichtung gemäß Figur 14 längs der Linie B --- B, wobei Figur 15a als Momentaufnahme in schematischer Form die Probenaufnahme demonstriert. Die Figur 15b zeigt die Vorrichtung in betriebsbereitem Zustand, indem der zur Trennung verwendete Hohlkörper jeweils oben und unten in ein Pufferreservoir 50 eintaucht.

Die in Figur 10 dargestellte bevorzugte Ausführungsform besteht aus zwei Heiz- oder Kühlvorrichtungen 1, 2 mit den dazugehörigen Temperaturen T₂ und T₁. Diese Heiz- bzw. Kühlvorrichtungen 1, 2 sind leitend mit Wärmereservoirs 4, 5 verbunden. Die Heiz- oder Kühlvorrichtungen sowie das Wärmereservoir weisen vorzugsweise zentrische Durchbohrungen auf. Durch diese Bohrungen ist ein Hohlkörper 6 vorzugsweise beidseitig durchdringend angeordnet. Der Hohlkörper 6 ist von einem Thermostatiermantel 7 allseitig umschlossen. Vorzugsweise befindet sich der Hohlkörper 6 im Thermostatiermantel 7 zentriert. Der Hohlkörper 6 enthält in seinem Lumen das zur Trennung verwendete Medium. Der Thermostatiermantel 7 besteht vorzugsweise aus wärmeleitendem Material, besonders bevorzugt einem Material, aus dem auch die Wärmereservoirs hergestellt sind. Die Temperaturniveaus 1, 2 werden vorzugsweise durch Peltierelemente, thermostatisierbare Flüssigkeitsbäder oder elektrische Heizungen 9 beheizt bzw. gekühlt. Am Ende der Trennstrecke ist eine Detektionseinheit 10 vorgesehen. Die Fig. 10a zeigt die Situation im Querschnitt längs der Linie A --- A. Der Zwischenraum zwischen der äußeren Wand des Hohlkörpers 6 und der inneren Wand des Thermostatiermantels 7 ist vorzugsweise durch eine viskose Flüssigkeit 8 ausgefüllt. Das Trennmedium füllt vollständig den Querschnitt des Hohlkörpers 6. Der Hohlkörper 6 ist vorzugsweise zylindrisch ausgebildet, besonders bevorzugt als Kapillare. Die an einer beliebigen Stelle der Trennstrecke herrschende Temperatur berechnet sich nach der Formel T = T₂ - (T₂ - T₁) x d₂/(d₁ + d₂). Dabei bedeutet d₁ der Abstand des Ortes von der Temperatur T₁ und d₂ der Abstand des Ortes von der Temperatur T₂, die jeweils an den Temperaturniveaus 2, 1 herrschen.

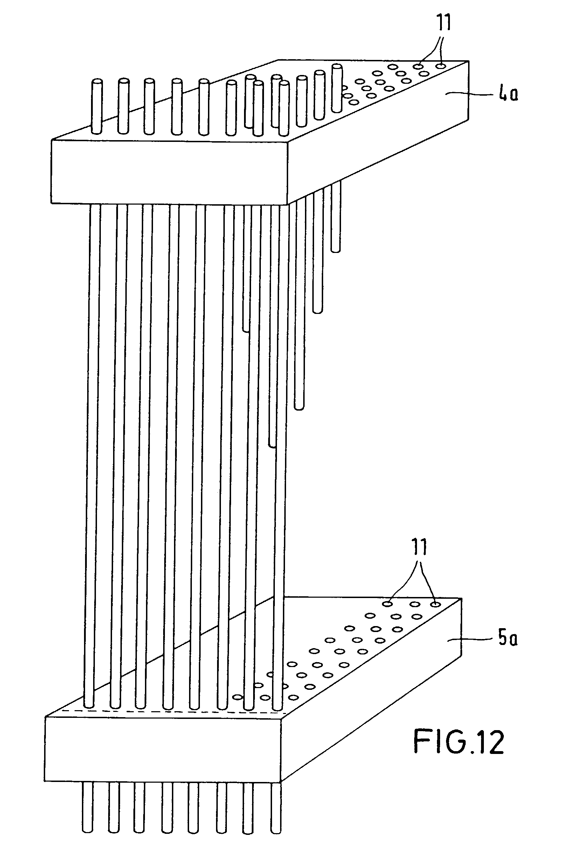

Die Fig. 11 zeigt eine bevorzugte Ausführungsform zur Durchführung des erfindungsgemäßen Verfahrens als einer von einem zeitlichen Temperaturgradienten überlagerten Elektrophorese. Sie besteht aus drei Temperaturniveaus 1, 2, 3, die vorzugsweise wiederum mit Wärmereservoirs verbunden sind. Die Komponenten Hohlkörper 6, Wärmeaustauschmantel 7, Detektionseinheit 10, Heiz- oder Kühlvorrichtung 9 sind ähnlich der Vorrichtung gemäß Fig. 10 ausgebildet. Lediglich der Thermostatiermantel 7 ist mit dem Wärmereservoir der Heiz- oder Kühlvorrichtung am Temperaturniveau 2 thermisch nicht leitend verbunden.

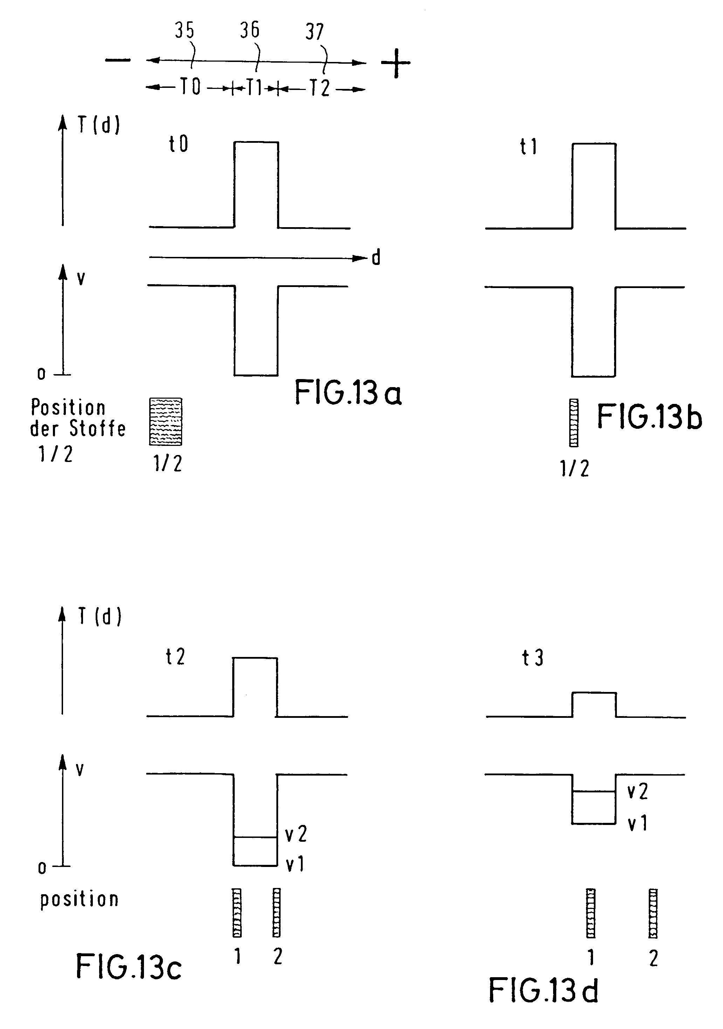

Die Figuren 13a bis 13d zeigen den Verfahrensablauf wie er beispielsweise mit einer Vorrichtung gemäß der Fig. 11 ausgeführt wird. Der Gradient des elektrischen Feldes, das zur Trennung verwendet wird, ist in Richtung der Abszisse aufgetragen und symbolisiert gleichzeitig beliebige Orte der zu durchlaufenden Trennstrecke. Auf der Ordinate sind einmal in positiver Richtung die Temperaturniveaus in Abhängigkeit vom Ort eingezeichnet (Fig. 13a). Die Wanderungsgeschwindigkeit der Komponenten der zu trennenden Probe ist durch Stufenfunktion unterhalb der Temperaturkurve dargestellt. Z.B. ist die Wanderungsgeschwindigkeit (V) bei T₁ (t₀) ungefähr gleich Null. Aufgetragen wird die Probe bei Temperaturniveau T₀, das in diesem Fall einfachheitshalber gleich dem Temperaturniveau T₂ am Ende der Elektrophorese-Trennstrecke sein soll. Nach Auftrag nehmen die Stoffe 1 und 2 das gepunktete Volumen in bezeichneter Position ein. Die Probe wandert längs des Feldgradienten in dem Trennmedium, bis sie das Temperaturniveau T₁ erreicht. Wenn beispielsweise die Probe aus Nukleinsäuren besteht, erfahren diese hier eine teilweise Denaturierung durch Aufweitung des Doppelstrangs in sogenannte "Loops". Dadurch bedingt wird die Wanderungsgeschwindigkeit reduziert. Die Probe wird aufkonzentriert an der Grenze zwischen T₀ und T₁. Diese Situation ist in Fig. 13b in Form des geringeren Volumens dargestellt (Zeitpunkt t₁). Die Temperatur des Temperaturniveaus T₁ wird nun abgesenkt als Funktion der Zeit. Die thermodynamisch stabilere Fraktion, in Fig. 13c als Fraktion Nr. 2 gekennzeichnet, schließt nun die Doppelhelix und gewinnt höhere Mobilität. Dadurch bedingt beginnt diese Fraktion in das Trennmedium zu wandern. Fig. 13d zeigt nun die Situation bei einem Zeitpunkt t₃, bei dem die Temperatur T so weit abgesunken ist, daß auch die thermodynamisch instabilere Probe, hier mit der Nr. 1 gekennzeichnet, mit der Wanderung durch das Trennmedium beginnt. Die Komponente Nr. 2 hat in dieser Zeit allerdings schon einen beträchtlichen Teil der Trennstrecke durchlaufen oder sie bei entsprechender Dimensionierung bereits verlassen, wo sie detektiert werden kann.

Diese Ausführungsform verwendet vorzugsweise eine sehr kurze Kapillare mit Trennmedium und läßt sich an drei thermisch voneinander getrennten Bereichen thermostatieren (T₀, T₁, T₂). Die an der Temperaturgrenze zwischen T₀ und T₁ stattfindende partielle Denaturierung, beispielsweise von Nukleinsäuren zur Bildung interner "Loops", kann durch entsprechende Reagenzien unterstützt werden.

An den Grenzen der einzelnen Temperaturniveaus kommt es jedoch über einen flüssigen Wärmetauscher zu unscharfen Temperaturgrenzen, so daß die Darstellung gemäß Fig. 12 nur als idealisierte Rechteckform von Temperatur und Wanderungsgeschwindigkeit gesehen werden soll. Um trotzdem eine möglichst scharfe Grenze der Temperaturniveaus zu gewährleisten, wird bevorzugt, die Temperaturniveaus T₂/T₁/T₀ nicht wärmeleitend zu verbinden.

Diese erfindungsgemäße Verfahrensweise ist auch mit flächigen Trennmedien realisierbar, die einseitig temperiert werden, wie es für einfache räumliche Temperaturgradienten in der deutschen Patentanmeldung P 36 22 591 beschrieben ist.

Die Fig. 12 zeigt eine bevorzugte Vorrichtung, die dadurch gekennzeichnet ist, daß Heiz- und Kühlvorrichtungen 1, 2 mit den entsprechenden Wärmereservoirs 4, 5 eine Vielzahl der Hohlkörper 6 aufnehmen können, indem die Vorrichtungen 1, 2 und 4, 5 blockartig - 4a, 5a - ausgestaltet sind und eine Vielzahl von Durchbohrungen 11 aufweisen, durch die die Hohlkörper 6 hinausragend, vorzugsweise endseitig hinausragend angeordnet sind. Diese Vorrichtung hat den Vorteil, daß sich mit einer Vorrichtung eine Großzahl von Analysen mit dem Ziel der Detektion von bekannten oder unbekannten Mutationen durchführen läßt. Die Vorrichtung gewährleistet, daß mehrere Proben gleichzeitig unter Temperierung durch verschiedene Thermostatierelemente, jedoch mit je einem gemeinsamen Heiz- oder Kühlsystem analysiert werden können. Eine bevorzugte Ausführungsform ist auf das Format Mikrotiter (96 Well) adaptiert im Sinne linearer Mehrkanalsysteme, vorzugsweise 8 oder 12, oder einem 96-Kanal-System. Als "Read out"-System finden vorzugsweise fluoreszenzmarkierte Nukleinsäuresonden Verwendung, die optisch durch handelsübliche Detektionssysteme am Anfang und/oder am Ende der Thermostatiervorrichtung als Funktion der Trenndauer und ortsfest registriert werden können. Damit lassen sich mit Hilfe von geeigneten Reagenzienkits Mutationen in genetischem Material automatisch auswerten.

Die thermische Äquilibrierung der jeweiligen Heiz- oder Kühlvorrichtungen kann homogen elektrisch über Heizdrähte, vorzugsweise jedoch über Peltierelemente erfolgen. Auch flüssige Heizvorrichtungen in Form thermostatierbarer Flüssigkeitsbäder kommen in Frage. Hierbei ist jedoch darauf zu achten, daß die mit den thermostatierbaren Ummantelungen der Kapillare verbundenen Heiz- oder Kühlvorrichtungen gegenüber allen Ummantelungen nahezu identische Temperaturen an den jeweiligen Übergangsstellen aufweisen. Dieses läßt sich durch eine symmetrisch gebaute Peltierheizung/-Kühlung realisieren oder im Falle der Flüssigtemperierung durch gegenläufige durchströmte Kanäle, wobei die Summe der Temperaturen gegenüberliegender Flüsse an jeder Kapillarposition nahezu identisch bleibt.

Die erfindungsgemäß ausgestaltete TemperaturgradientenGelelektrophorese eignet sich insbesondere auch für eine Vorgehensweise, bei der ein Temperaturgradient nicht räumlich dimensioniert ist, sondern zeitlich variabel bzw. zeitlich gradientenförmig aufgebaut wird. Darunter ist zu verstehen, daß ein Heiz- oder Kühlreservoir mit einem zeitlich definierten Temperaturprogramm geregelt wird, wodurch sich die Mobilitäten der zu trennenden Moleküle letztlich als Funktion der Zeit steuern lassen. So läßt sich zum Beispiel eine offene zirkuläre Nukleinsäure oder auch eine partiell denaturierte doppelsträngige Nukleinsäure bei hohen Temperaturen im Gel quasi "arretieren" und erst nach Ablauf einer bestimmten Zeit durch Senken der Temperatur nach reversibler Strukturrückbildung in dem Trennmedium mobilisieren. Dies gilt für die Trennung in Kapillaren und auf flächigen Trägern. Der Vorteil dieser Technik liegt darin, daß hierbei äußerst kurze Trennstrecken realisiert werden können.

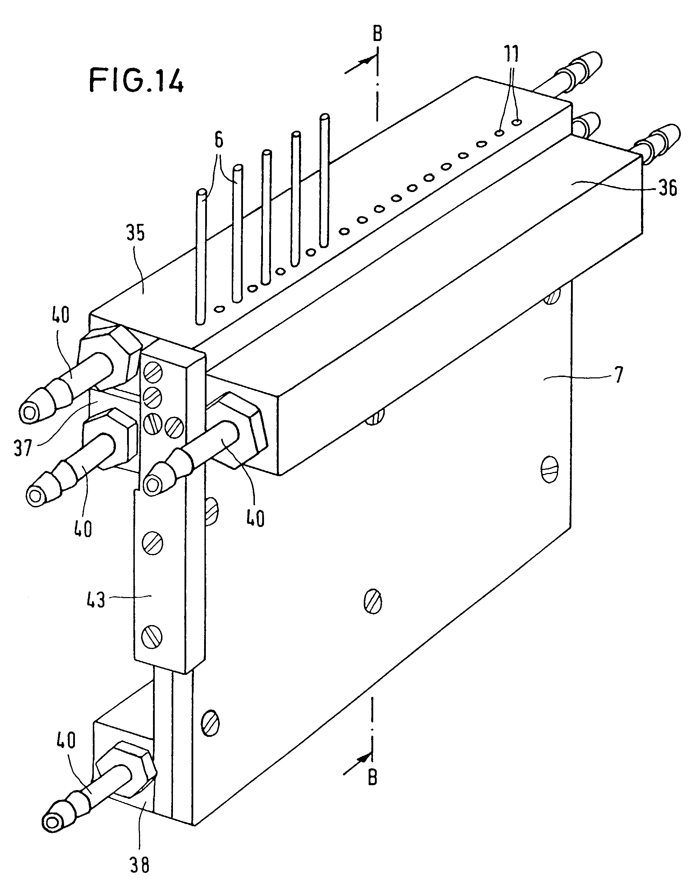

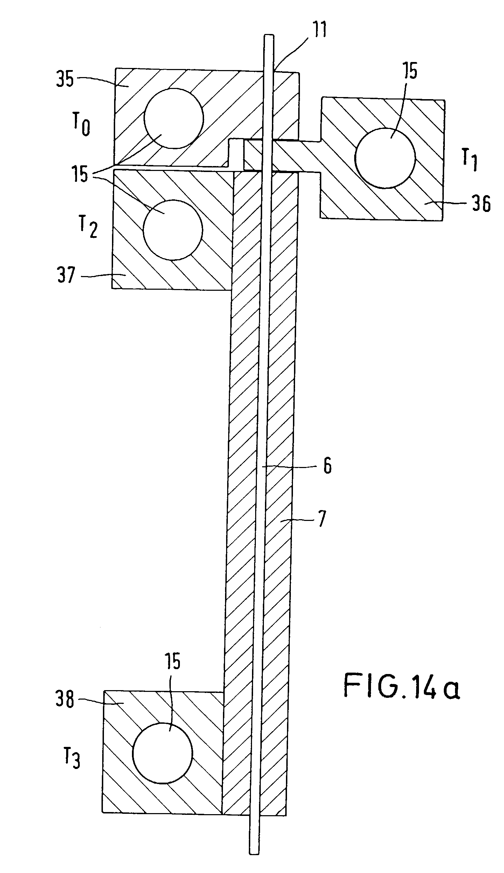

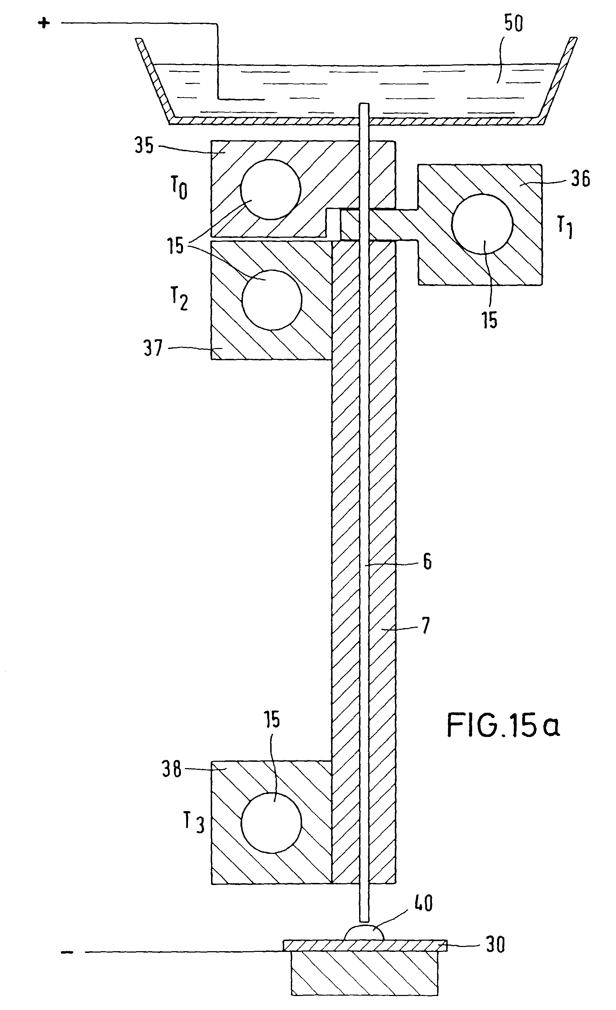

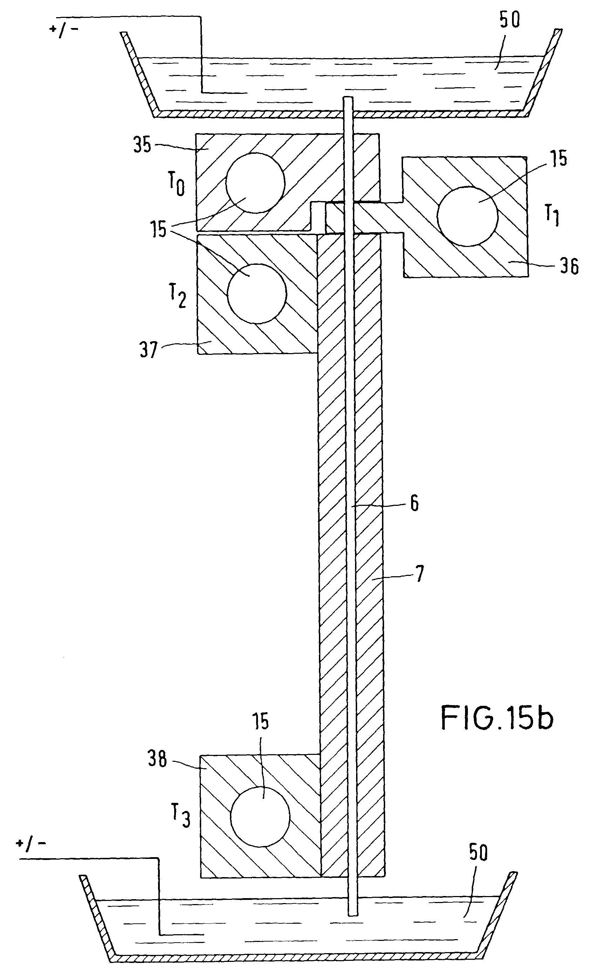

Die schematischen Figuren 15a und b zeigen eine bevorzugte Vorrichtung zur Durchführung des erfindungsgemäßen Verfahrens, in welches gemäß Figur 14 12 Proben simultan analysiert werden können. Es ist erkennbar, daß insgesamt 4 Temperaturniveaus T₀ bis T₃ variabel geregelt werden können. Mit dieser bevorzugten Vorrichtung lassen sich sowohl räumliche, zeitliche als auch Kombinationen aus raumlichen und zeitlichen Gradienten aufbauen. Diese erfindungsgemäße Vorrichtung ist besonders geeignet, im Laboratorium die Parameter zur Trennung der zu anlysierenden Proben zu optimieren.

Die Figur 14 zeigt eine bevorzugte Ausführungsform der erfindungsgemäßen Vorrichtung. Es sind insgesamt 4 regelbare Heiz- bzw. Kühlvorrichtungen (35 bis 38) vorhanden. Die Temperaturniveaus T₀ bis T₃ sind unabhängig voneinander regelbar. Die Temperatur wird durch Einleiten entsprechend temperierter Flüssigkeiten, durch die Zuläufe 40, in die hier als Metallblöcke ausgestalteten Temperaturreservoirs eingestellt. Auf der gegenüberliegenden Seite der Zuflüsse 40 werden die zur Temperierung verwendeten Flüssigkeiten durch entsprechende Abläufe (hier nicht gezeichnet) abgeleitet. Der das Temperaturniveau 35 bildende Metallblock ist mit Durchbohrungen versehen, in welchen die zur Trennung verwendeten Hohlkörper 6 durchgeschoben werden können. Die den Thermostatiermantel bildende Metallplatte 7 ist aus zwei Teilen, die vorzugsweise verschraubt sind, aufgebaut. In dieser Platte befinden sich an jeweils gegenüberliegenden Positionen eingefräste Nuten, die auf den Durchmesser des Hohlkörpers 6 abgestimmt sind. Die Metallblöcke 35 bis 38, welche die entsprechenden Temperaturniveaus bereitstellen, sind mittels eines Verbindungsstücks 43 durch Schrauben mit dem Thermostatiermantel 7 verbunden.

Die Figur 14a zeigt einen Querschnitt der in Figur 14 gezeichneten Vorrichtung längs der Linie B --- B. Der zur Trennung dienende Hohlkörper 6 durchdringt die den Thermostatiermantel bildenden Hohlkörper 7 vollständig. Vorzugsweise befindet sich im Zwischenraum, der zwischen Hohlkörper 6 und Thermostatiermantel 7 gebildet wird, eine viskose Flüssigkeit. Die Metallblöcke 37 und 38 sind thermisch leitend durch direkten Kontakt der planaren Seitenflächen mit dem Thermostatiermantel 7 verbunden. Vorzugsweise sind die Blöcke 37, 38 und der Thermostatiermantel 7 aus dem selben Material gefertigt. In einer besonderen Ausführungsform sind diese Elemente der erfindungsgemäßen Vorrichtung einstückig. Die das Temperaturniveau T₀, T₁ aufbauenden Metallblöcke sind beispielsweise einstückig mit dem den Hohlkörper 6 umfassenden Thermostatiermantel. Die die Temperaturniveaus T₀, T₁ aufbauenden Metallblöcke 35, 36 sind durchbohrt. Die Bohrungen in den verschiedenen Metallblöcken und dem Thermostatiermantel werden zur Deckung gebracht, so daß der zur Trennung verwendete Hohlkörper 6 durch die aus den einzelnen Elementen 35, 36, 37 und 38 aufgebaute Vorrichtung hindurchgeschoben werden kann. Die Metallblöcke 35 bis 38 sind mit den Durchbohrungen 15 (Querschnitt) versehen, durch welche die zum Aufbau des jeweiligen Temperaturniveaus verwendete Flüssigkeit hindurchströmt.

Im folgenden wird beschrieben, wie das in der Figurenbeschreibung zu Figur 2 und 3 beschriebene Gemisch aus markierten Hybriden, die einen Homoduplex und einen Heteroduplex (Einbasen-mismatch) repräsentieren, getrennt wird. Die zwei in der Figurenbeschreibung zu Figur 2 und 3 beschriebenen klonierten Inserts (Wildtyp und IVS-1-6) wurden in Form ihrer EcoR1/Bam H1 verdauten Plasmide (je 45 ng) gemischt. Ihnen wurde in limitierter Menge (9 ng bezogen auf Plasmid) das radioaktiv markierte Insert beigemischt, wobei der Radioaktivitätsmarker durch 32P-dATP, dCTP, dGTP, dTTP-Einbau mit Polymerase-l Klenow-Fragment erfolgt. Die spezifische Aktivität betrug ca. 10⁶ cpm/pMol Schnittstelle. Die Mischung wurde in 55 µl Puffer 10 mM Tris, 1 mM EDTA pH 7,5 bei 98°C für 2 Minuten denaturiert und nach Einstellen auf 250 mM NaCl bei 50°C für eine Stunde renaturiert. Die DNA wurde mit 2,5 Vol. Ethanol 30 Minuten bei -20°C gefällt, mit 80 % Ethanol gewaschen und getrocknet. Die Probe wurde in 0,01 x TBE Bromphenolblau aufgenommen. 13.000 cpm in 3 µl wurden auf einer planen Platinelektrode (Fig. 15a 30) als Tropfen 40 vorgelegt. Der Probenauftrag erfolgte elektrophoretisch nach Eintauchen der Kathoden-seitigen Kapillare durch Anlegung einer Spannung von 100 V gegen die geerdete Platinkathode 30 (Fig. 15a) für je 2 Minuten. Da der TBE-Puffer (89 mM Tris, 89 mM Borsäure, 2,5 mM EDTA, pH 8,3) in Richtung hoher pH-Werte eine hohe Pufferkapazität aufweist, wird die Probe trotz niedriger Pufferkonzentration (0,01 x TBE) nicht um mehr als eine pH-Einheit alkalischer (pH 8,3 bis 9,3). Ca. 50 % der markierten Nukleinsäure wird auf diese Weise vom Gel aufgenommen.

Als Kapillaren wurden gefüllte Glaskapillaren mit 5 % Polyacrylamid-Gel und einem Innendurchmesser von 0,45 mm verwendet. Die Pufferbedingungen waren mit 0,1 x TBE, 4 M Harnstoff gewählt. Die Figur 15b zeigt schematisch die erfindungsgemäße Vorrichtung beim Elektrophoresebetrieb. nach erfolgter Probenaufnahme werden beide Enden der Kapillaren 6 in Kontakt mit je 100 ml Pufferreservoir 50 gebracht.
a) Die Probe wurde in einem räumlichen Gradienten aufgetrennt (T₃ = 30°C, T₂=70°C).
Tabelle 1 zeigt die Differenz der Wanderungsstrecken des Homoduplexes verglichen mit der jeweiligen Wanderungsstrecke des Heteroduplexes in cm. Im erwarteten Temperaturintervall 40°C bis 50°C ergibt sich die experimentell gewünschte Auftrennung der Signale.

| Elektrophoresedauer (min.) | Differenz der Kapillargel-Position (mm) von Wildtyp Fragment und IVS-1-6-Fragment |
|---|---|
| 30 | 0 |
| 42 | 0 |
| 54 | 0 |
| 66 | 0 |
| 78 | 2 |
| 90 | 4 |
| 102 | 6 |
| 114 | 6 |
| 126 | 7 |
| 138 | 7 |
| 150 | 9 |
| 162 | 11 |

Die Daten beruhen auf Einzelmessungen in getrennten Kapillaren.
b) Analog der Vorgehensweise der Figuren 13a bis d wurde die Probe auf der Seite T₀ aufgetragen. (T = T₂ (37) = T₃ = 30°C) und die Trennung über die 4 mm Trennstrecke mit der Temperatur T₁ durchgeführt. Nur die Proben, die T₁ im Temperaturintervall (40 < T₁ < 50) passiert haben, werden aufgetrennt.

Während der Elektrophorese wurde erfindungsgemäß das Temperaturniveau von T₁ linear abgesenkt. Die Proben, die T₁ knapp unterhalb der Temperatur erreichen, bei der die Dissoziation in die Einzelstränge erfolgt (50°C), werden im Segment T₁ (36) (4 mm Trennstrecke) in Banden in einem relativen Abstand von bis zu 1 cm getrennt.

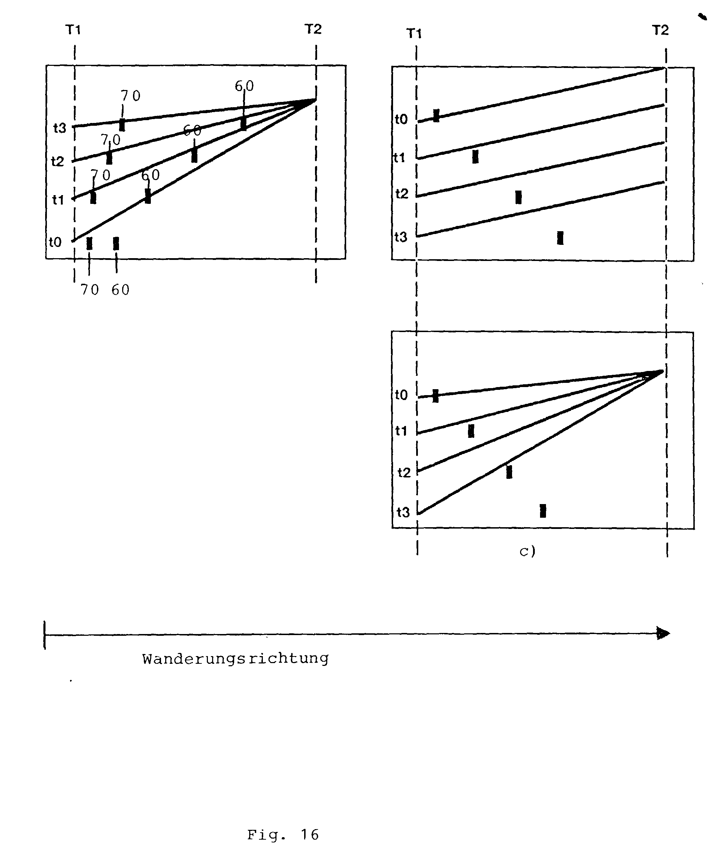

Die Kombination räumlicher und zeitlicher Gradienten der Temperatur, wie sie mit einem statisch chemischen Gradientensystem nicht erreicht werden kann, ist für einige Anwendungen von großer praktischer Bedeutung. Zwei Anwendungen seien exemplarisch dargestellt. Sie werden in Figur 16 schematisch erläutert.
a) Fragmente sehr unterschiedlicher nativer Wanderungsgeschwindigkeit, z.B. infolge stark differierender Größen der Fragmente, sind gemeinsam nur schwierig zu analysieren. Mitunter unterliegt das schnellwandernde kleine Fragment 60 (Fragmente und ihre jeweiligen Gelpositionen sind als kleine Balken repräsentiert) bereits der Strangtrennung, während das große Fragment 70 die Temperatur beginnender Denaturierung noch nicht erreicht hat. Erfindungsgemäß wird die hohe Temperatur T₂ so eingestellt, daß noch keine Trennung des stabilsten Doppelhelix-Segments bzw. der G:C Klammer eintritt. Die Temperatur T₁ wird jedoch während der Elektrophorese von niedriger Temperatur auf stetig höhere Temperatur geregelt, höchstens bis T₁ = T₂ erreicht ist. Auf diese Weise durchläuft jedes Molekül den Temperaturgradienten, unabhängig von seiner Wanderungsgeschwindigkeit bzw. Größe.
b) Renaturierungsexperimente, wie sie in der Figur 16b und 16c schematisch beschrieben sind, ergeben nur dann scharfe Bandensignale, wenn nicht allein ein räumlicher Temperaturgradient durchlaufen wird. In diesem Fall kommt es nämlich zu einem unerwünschten Effekt der Bandenverbreiterung bei der Rückfaltung, der umso störender ist, je steiler die Denaturierungskurve verläuft. In diesem Fall wird die Front der Bande gegenüber dem Bandenende stark beschleunigt, da rückseitig eine niedrigere Temperatur herrscht. Im Ergebnis wird die Bande diffus. Der Effekt ist hingegen sehr erwünscht, wenn umgekehrt die Bandenfront in Laufrichtung gesehen relativ bei höheren Temperaturen läuft. Erfindungsgemäß läßt sich dies auch bei Renaturierungsexperimenten erreichen durch Kombination räumlicher und zeitlicher Gradienten. Anstelle einer Renaturierung in einem linearen T-Gradienten, wie er z.B. mit einer Vorrichtung gemäß Figur 1 durchgeführt werden kann, kann die Probe in einem relativ steigenden Gradienten zwischen T₁ und T₂ (Figur 10) in den Zeiten t₀ bis t₃ (Figur 16b und c) der Elektrophorese unterworfen und analysiert werden, wobei jedoch beide Temperaturen gemeinsam (Figur 16b) oder nur T₁ (Figur 16c) in einem Maße herabgesetzt werden, daß die Probe mit der Laufzeit einer niedrigeren Temperatur ausgesetzt ist, die Bandenfront jedoch immer eine höhere Temperatur hat als ihre Rückseite. Auf diese Weise tritt erfindungsgemäß eine Bandenschärfung ein.

Das erfindungsgemäße Verfahren und die erfindungsgemäße Vorrichtung können sowohl zur analytischen Erfassung und quantitativen Erfassung als auch zur Präparation von Komponenten aus Stoffgemischen verwendet werden. Die Analyse und Präparation können an vielen Proben simultan durchgeführt werden; Detektion und Auswertung können automatisch erfolgen. Insbesondere eignen sich Verfahren und Vorrichtung zur Präparation und Analyse von Viroiden, viralen Nukleinsäuren, Satelliten-RNA, zur Analyse von Mutationen in Nukleinsäuren, zur Analyse von Mutationen in Proteinen und zur Analyse von Protein-Nukleinsäure-Komplexen.

Die TGGE erweist sich zur Präparation von Varianten als besonders vorteilhaft, da die direkte Sequenzierung von Varianten möglich wird ohne vorherige Klonierung. Dies wird ermöglicht durch Elution geringster Mengen einer spezifischen Variante, die einer enzymatischen Amplifikation und nachfolgender Sequenzierung unterworfen wird. Solche Arbeitsweisen werden in Zukunft an Bedeutung gewinnen, da durch Sicherheitsauflagen das Arbeiten mit sonst zur Amplifikation verwendeten Vektoren und das Arbeiten mit rekombinanten Organismen erschwert wird.

## Patentansprüche

1. Verfahren zur quantitativen und qualitativen Detektion von Mutanten oder spezifischen Gensequenzen, wobei einem Gemisch differierender Sequenzen, deren eine Konzentration bekannt sein kann, im Unterschuß ein Marker-Nukleinsäuremolekül zugesetzt wird, dessen Sequenz mit der Sequenz einer der vorgenannten Sequenzen mit Ausnahme der Markierung identisch ist und dieses Gemisch mindestens einem Denaturierungs- und Renaturierungszyklus unterworfen und analysiert wird, wobei die nach einem Denaturierungs-/Renaturierungszyklus gebildeten Hybride mittels Temperaturgradientengelelektrophorese (TGGE) getrennt werden und deren relative Signal intensitäten gemessen werden.

2. Verfahren nach Anspruch 1, wobei die Sequenz der markierten Nukleinsäure mit der Sequenz der Nukleinsäure, deren Konzentration bekannt ist, identisch ist, mit Ausnahme der Markierung.

3. Verfahren nach einem der Ansprüche 1 und/oder 2, wobei die zu analysierenden Nukleinsäuren durch enzymatische Amplifikation erhalten worden sind.

4. Verfahren nach Ansprüchen 1 bis 3 zur qualitativen und quantitativen Detektion von Mutationen von Nukleinsäuren durch Analyse des durch Hybridisierung des die Mutation aufweisenden Nukleinsäurefragments (Mutante) mit dem diese Mutation nicht aufweisenden Nukleinsäurefragment (Wildtyp) gewonnenen Heteroduplexes, wobei das die Mutation tragende, zu untersuchende Nukleinsäurefragment so gewählt wird, daß die Mutation in einer thermodynamisch instabilen Region des Heteroduplexes liegt.

5. Verfahren nach mindestens einem der Ansprüche 1 bis 4, wobei die Auswahl der die Mutation enthaltenden thermodynamisch instabilen Region durch Berechnung erfolgt.

6. Verfahren nach mindestens einem der Ansprüche 1 bis 5, wobei das zu untersuchende Nukleinsäurefragment so gestaltet wird, daß die Mutation in der thermodynamisch instabilsten Region liegt.

7. Verfahren nach einem der Ansprüche 1 bis 6, wobei die thermodynamisch stabilere Region des Nukleinsäurefragments endständig Oligo-G- und/oder -C-Nukleotide trägt.

8. Verfahren nach Anspruch 7, wobei die Anzahl der G- und/oder C-Nukleotide 20 bis 30 beträgt.

9. Verfahren nach einem der Ansprüche 1 bis 8, wobei die Nukleinsäuren durch Polymerase Chain Reaction (PCR) amplifiziert werden.

10. Verfahren nach einem der Ansprüche 1 bis 9, wobei die beiden verwendeten Oligonukleotide in unmittelbarer Nachbarschaft zur nachzuweisenden Mutation an die zu amplifizierende DNA hybridisieren.

11. Verfahren nach einem der Ansprüche 1 bis 10, wobei die zu untersuchenden Heteroduplices nach Probenvorbereitung einer Temperaturgradienten-Gelelektrophorese unterzogen werden.

12. Verfahren nach Anspruch 11, wobei der Temperaturgradient senkrecht zur Richtung des elektrischen Feldes verläuft.

13. Verfahren nach Anspruch 11, wobei der Temperaturgradient parallel zur Feldrichtung des elektrischen Feldes verläuft.

14. Verfahren nach Anspruch 13, wobei der Temperaturgradient zeitlich variiert wird.

15. Verfahren nach Anspruch 14, wobei der Temperaturgradient von einem höheren Temperaturniveau kathodenseitig des elektrischen Feldes ausgeht, das über dem Schmelzpunkt der thermodynamisch instabilen Region der Heteroduplices liegt, und zeitlich in Richtung zum Niveau des anodenseitig liegenden tieferen Temperaturniveaus reguliert wird.

16. Verfahren nach Anspruch 13, wobei das anodenseitige Temperaturniveau des statischen Temperaturgradienten höher liegt als das Temperaturniveau der Kathodenseite.

17. Verfahren nach Anspruch 13, wobei das kathodenseitige Temperaturniveau des statischen Temperaturgradienten höher liegt als das Temperaturniveau der Anodenseite.

18. Verfahren nach einem der Ansprüche 1 bis 17, wobei die die Mutation tragende Nukleinsäure unter Verwendung eines Oligonukleotids mit 5'-terminaler Affinitätsgruppe amplifiziert wird, an einem zu den Affinitätsgruppen der Oligonukleotide mit 5'-terminalen Affinitätsgruppen an fester Phase fixierten affinen Material gebunden wird, sodann in Gegenwart gewünschtenfalls markierter, die Mutation nicht aufweisender Nukleinsäure-Einzel- oder -Doppelstränge mindestens einem Denaturierungs-/Renaturierungszyklus unterworfen wird, anschließend eluiert und danach dem Trennungsverfahren unterworfen wird.

19. Verfahren nach Anspruch 18, wobei das zu der Affinitätsgruppe des Oligonukleotids mit 5'-terminaler Affinitätsgruppe affine Material ein polymerer Träger ist, der ein Chelat aus einem Chelatbildner und einem Übergangsmetallion enthält.

20. Verfahren nach Anspruch 19, wobei das zu der Affinitätsgruppe des Oligonukleotids affine Material ein polymerer Träger ist, der ein Chelat aus kovalent an den Träger gebundener Nitrilotriessigsäure und Nickel²⁺ enthält.

21. Verfahren nach Anspruch 18, wobei das zu der Affinitätsgruppe des Oligonukleotids mit 5'-terminaler Affinitätsgruppe affine Material ein polymerer Träger ist, der kovalent gebundenes Avidin oder Streptavidin aufweist.

22. Verfahren nach einem der Ansprüche 18 bis 21, wobei der polymere Träger eine Membran oder ein partikelförmiges Material ist.

23. Verwendung eines Mittels, bestehend aus einem Gemisch von Reagentien wie einer oder mehrerer markierter Nukleinsäuresonden, einer teilweise oder vollständig homologen Standardnukleinsäure in nicht markierter Form sowie einem Hybridisierungspuffer, der im Temperaturbereich zwischen 0 und 100°C Denaturierung und Renaturierung von Doppelstrangstrukturen erlaubt, zur Durchführung des Verfahrens nach mindestens einem der Ansprüche 1 bis 22.

24. Verwendung nach Anspruch 23, wobei das Mittel besteht aus einem Gemisch von mindestens einer markierten Nukleinsäuresonde, einer teilweise oder vollständig homologen Standardnukleinsäure in nicht markierter Form sowie einem Hybridisierungspuffer, der im Temperaturbereich zwischen 0 und 100°C Denaturierung und Renaturierung von Doppelstrangstrukturen erlaubt.

25. Verwendung nach Anspruch 24, wobei das im Mittel enthaltene Gemisch einen Festphasenträger nach einem der Ansprüche 18 bis 22 enthält, mit dessen Hilfe amplifizierte Segmente direkt dem Reaktionsgemisch entzogen werden können.

## Claims

1. A method for the quantitive and qualitative detection of mutants or specific gene sequences wherein to a mixture of different sequences, the concentration of one of which may or may not be known, a subequivalent amount of a labeled nucleic acid molecule is added the sequence of which is identical, with the exception of the label, with the sequence of one of the above sequences and the resulting mixture is subjected to at least one denaturing and renaturing cycle and then analyzed, wherein the hybrids formed after each denaturing and renaturing cycle are separated by means of temperature gradient gel electrophoresis (TGGE) and their relative signal intensities measured.

2. The method according to claim 1, wherein the sequence of said labeled nucleic acid is identical, with the exception of the label, with the sequence of the nucleic acid the concentration of which is known.

3. The method according to any of claims 1 and/or 2, wherein the nucleic acids to be analyzed have been obtained by enzymatic amplification.

4. The method according to claims 1 to 3 for the qualitative and quantitive detection of mutants of nucleic acids by analyzing the heteroduplex obtained by hybridizing the nucleic acid fragment having the mutation (mutant) with the nucleic acid fragment lacking such mutation (wild type) wherein said nucleic acid fragment bearing the mutation which is to be examined is selected such that the mutation lies in a thermodynamically unstable region of said heteroduplex.

5. The method according to at least one of claims 1 to 4, wherein the selection of the thermodynamically unstable region containing the mutation is made by computing.

6. The method according to at least one of claims 1 to 5, wherein the nucleic acid fragment to be examined is selected such that the mutation lies in the most thermodynamically unstable region.

7. The method according to any of claims 1 to 6, wherein the thermodynamically more stable region of the nucleic acid fragment bears terminal oligo-G and/or oligo-C nucleotids.

8. The method according to claim 7, wherein the number of said G and/or C nucleotids is from 20 to 30.

9. The method according to any of claims 1 to 8, wherein said nucleic acids have been amplified by polymerase chain reaction (PCR).

10. The method according to any of claims 1 to 9, wherein the two oligonucleotides employed will hybridize to the DNA to be amplified at a site adjacent to the mutation to be detected.

11. The method according to any of claims 1 to 10, wherein the heteroduplexes to be examined are subjected to temperature gradient gel electrophoresis following sample preparation.

12. The method according to claim 11, wherein said temperature gradient is in a direction perpendicular to that of the electric field.

13. The method according to claim 11, wherein said temperature gradient is in a direction parallel to that of the electric field.

14. The method according to claim 13, wherein said temperature gradient is varied with time.

15. The method according to claim 14, wherein on the cathode side of the electric field said temperature gradient starts at a higher temperature level, which is above the melting point of the thermodynamically unstable region of the heteroduplexes, and is regulated with time to approach the lower temperature level of the anode side.

16. The method according to claim 13, wherein the temperature level of the anode side of the static temperature gradient is higher than the temperature level of the cathode side.

17. The method according to claim 13, wherein the temperature level of the cathode side of the static temperature gradient is higher than the temperature level of the anode side.

18. The method according to any of claims 1 to 17, wherein the nucleic acid bearing the mutation is amplified using an oligonucleotide having a 5'-terminal affinity group, is bound on a material fixed on a solid phase having affinity to the affinity groups of said oligonucleotides having 5'-terminal affinity groups, is then subjected to at least one denaturing/renaturing cycle in the presence of optionally labeled nucleic acid single or double strands lacking said mutation, is subsequently eluted and then subjected to the separation procedure.

19. The method according to claim 18, wherein said material having affinity to the affinity group of said oligonucleotide having a 5'-terminal affinity group is a polymeric support containing a chelate of a chelating agent and a transition metal ion.

20. The method according to claim 19, wherein said material having affinity to the affinity group of said oligonucleotide is a polymeric support containing a chelate of nitrilotriacetic acid bound to said support and nickel²⁺.

21. The method according to claim 18, wherein said material having affinity to the affinity group of said oligonucleotide having a 5'-terminal affinity group is a polymeric support to which avidin or streptavidin is covalently bound.

22. The method according to any of claims 18 to 21, wherein said polymeric support is a membrane or a particulate material.

23. Use of an agent consisting of a mixture of reagents, such as one or more labeled nucleic acid probes, a standard nucleic acid in non-labeled form which is partially or entirely homologous thereto, and a hybridization buffer allowing denaturation and renaturation of double-stranded structures in the temperature range of between 0 and 100°C, for performing the method according to at least one of claims 1 to 22.

24. The use according to claim 23, wherein said agent consists of a mixture of at least one labeled nucleic acid probe, a standard nucleic acid in non-labeled form which is partially or entirely homologous thereto, and a hybridization buffer allowing denaturation and renaturation of double-stranded structures in the temperature range of between 0 and 100°C.

25. The use according to claim 24, wherein said mixture contained in said agent contains a solid-phase support as defined in any of claims 18 to 22 by means of which amplified segments can be directly withdrawn from the reaction mixture.

## Revendications

1. Procédé pour la détection quantitative et qualitative de mutants ou de séquences de gènes spécifiques, selon lequel on ajoute, à un mélange de séquences différentes dont une concentration peut être connue, une quantité déficitaire de molécule d'acide nucléique de marqueur dont la séquence est identique à la séquence d'une des séquence précitées, à ----- l'exception du marquage, on soumet ce mélange à au moins un cycle de dénaturation et de renaturation et on l'analyse, les hybrides formés après un cycle de dénaturation/renaturation étant séparés à l'aide d'une électrophorèse sur gel à gradient de température (TGGE), et on mesure l'intensité relative de leurs signaux.

2. Procédé selon la revendication 1, dons lequel la séquence de l'acide nucléique marqué est identique à la séquence de l'acide nucléique dont la concentration est connue, à l'exception du marquage.

3. Procédé selon l'une des revendications 1 et/ou 2, dans lequel les acides nucléiques à analyser sont obtenus par amplification enzymatique.

4. Prcocédé selon les revendications 1 à 3 pour la détection quantitative et quantitative de mutations d'acides nucléiques par analyse de l'hétéroduplex obtenu par hybridation du fragment d'acide nucléique présentant la mutation (mutant) avec le fragment d'acide nucléique ne présentant pas cette mutatuion (type sauvage), dans lequel le fragment d'acide nucléique à analyser, qui porte la mutation, est choisi de telle façon que la mutation est située dans une région de l'hétéroduplex qui est instable du point de vue thermodynamique.

5. Procédé selon au moins une des revendications 1 à 4, dans lequel le choix de la région contenant la mutation et qui est instable du point de vue thermodynamique est effectué à ----- l'aide d'un calcul.

6. Procédé selon au moins une des revendications 1 à 5, dans lequel le fragment d'acide nucléique à analyser est agencé de telle façon que la mutation est située dans la région la plus instable du point de vue thermodynamique.

7. Procédé selon au moins une des revendications 1 à 6, dans lequel la région du fragment d'acide nucléique qui est relativement stable du point de vue thermodynamique porte des oligo-G-nucléotides et/ou des oligo-C-nucléotides en position terminale.

8. Procédé selon la revendication 7, dans lequel le nombre des G-nucléotides et/ou des C-nucléotides est de 20 à 30.

9. Procédé selon l'une des revendications 1 à 8, dans lequel les acides nucléiques sont amplifiés au moyen d'une réaction en chaîne de la polymérase (*Polymeraso Chain Reacttion* - PCR).

10. Procédé selon l'une des revendications 1 à 9, dans lequel les deux oligonucléotides utilisés s'hybrident à proximité directe pour donner la mutation à détecter sur l'ADN à amplifier.

11. Procédé selon l'une des revendications 1 à 10, dans lequel, après la préparation d'échantillons, les hétéroduplex à analyser sont soumis à une électrophorèse sur gel à gradient de température.

12. Procédé selon la revendication 11, selon lequel le gradient de température est orienté perpendiculairement à la direction du champ électrique.

13. Procédé selon la revendication 11, selon lequel le gradient de température est orienté parallèlement à la direction du champ électrique.

14. Procédé selon la revendication 13, dans lequel le gradient de température varie avec le temps.

15. Procédé selon la revendication 14, dans lequel le gradient de température part d'un niveau de températures élevées du côté cathode du champ électrique, qui est situé au-dessus du point de fusion de la région des hétéroduplex qui est instable du point de vue thermodynamique, et est régulé avec le temps pour atteindre le niveau des températures inférieures, lequel est situé du côté anode.

16. Procédé selon la revendication 13, dans lequel le niveau de températures du gradient statique de températures du côté anode est supérieur au niveau de températures du côté cathode.

17. Procédé selon la revendication 13, dans lequel le niveau de températures du gradient statique de températures du côté cathode est supérieur au niveau de températures du côté anode.

18. Procédé selon l'une des revendications 1 à 17, dans lequel l'acide nucléique portant la mutation est amplifié grâce à l'utilisation d'un oligonucléotide avec un groupe d'affinité on position terminale 5', il est lié à un matériau fixé on phase solide ayant une affinité pour les groupes d'affinité des oligonucléotides avec des groupes en position terminale 5', ensuite, il est soumis à au moins un cycle de dénaturation/renaturation on présence de simples brins ou de double brins d'acide nucléique, éventuellement marqués et qui ne présentent pas la mutation, et, enfin, il est élué, puis soumis au processus de séparation.

19. Procédé selon la revendication 18, dans lequel le matériau ayant une affinité pour le groupe d'affinité de l'oligonucléotide avec un groupe d'affinité en position terminale 5' est un support polymère qui contient un composé chélaté formé d'un agent de chélation et d'un ion d'un métal de transition.

20. Procédé selon la revendication 19, dans lequel le matériau ayant une affinité pour le groupe d'affinité de l'oligonucléotide est un support polymère qui contient un composé chélaté formé d'acide nitrilotriacétique, fixé sur le support au moyen d'une liaison covalente, et de nickel²⁴.

21. Procédé selon la revendication 18, dans lequel le matériau ayant une affinité pour le groupe d'affinité de l'oligonucléotide avec un groupe d'affinité en position terminale 5' est un support polymère qui présente de l'avidine ou de la streptavidine liée par covalence.

22. Procédé selon l'une des revendications 18 à 21, dans lequel le support polymère est une membrane ou un matériau en particules.

23. Utilisation d'un agent formé d'un mélange de réactifs, comme une ou plusieurs sondes d'acides nucléiques marqués, un acide nucléique standard partiellement ou complètement homologue sous une forme non marquée, ainsi qu'un tampon d'hybridation qui permet la dénaturation et la renaturation de structures à double-brin à une température comprise entre 0 et 100°C, pour la mise on oeuvre du procédé selon au moins une des revendications 1 à 22.

24. Utilisation selon la revendication 23, dans lequel ledit agent est formé d'un mélange d'au moins une sonde d'acide nucléique marqué, d'un acide nucléique standard partiellement ou complètement homologue sous une forme non marquée, ainsi que d'un tampon d'hybridation qui permet la dénaturation et la renaturation de structures à double-brin à une température comprise entre 0 et 100°C.

25. Utilisation selon la revendication 24, dans lequel ledit mélange contenu dans l'agent contient un support de phase solide selon l'une des revendications 18 à 22, à l'aide duquel les segments amplifiés peuvent être retirés directement du mélange de réaction.
